# EUROPÄISCHE PATENTSCHRIFT

(11) **EP 0 878 548 B1**
(45) Veröffentlichungstag und Bekanntmachung des Hinweises auf die Patenterteilung: **01.09.2004**
(21) Anmeldenummer: 98108721.6
(22) Anmeldetag: 13.05.1998
(51) Int. Cl.: C12P 17/18, C12P 13/02, C07C 233/23, C07C 231/18, C07C 231/20

(54) **Mehrstufiges Verfahren zur Herstellung von (1S,4R)- und/oder (1R,4S)-4-(2-Amino-6-Chlor-9-H-purin-9-yl)-2-cyclopenten-1-methanol**
Multistep process for the preparation of (1S,4R)- and/or (1R,4S)-4-(2-Amino-6-Chlor-9-H-purin-9-yl)-2-cyclopentene-1-methanol
Procédé à étapes multiples pour la préparation de (1S,4R)- et/ou (1R,4S)-4-(2-amino-6-chloro-9-H-purine-9-yl)-2-cyclopentene-1-methanol

(30) Priorität: 13.05.1997 CH 111697; 27.11.1997 CH 274097
(43) Veröffentlichungstag der Anmeldung: 18.11.1998
(73) Patentinhaber: Lonza AG, 3930 Visp (CH)
(72) Erfinder: Bernegger, Christine, Dr., 3985 Münster, Kanton Wallis (CH); Urban, Eva-Maria, 3930 Visp, Kanton Wallis (CH); Birch, Olwen Mary, Dr., 3930 Visp, Kanton Wallis (CH); Burgdorf, Kurt, 3911 Ried-Brig, Kanton Wallis (CH); Brux, Frank, 3942 Raron, Kanton Wallis (CH); Etter, Kay-Sara, 3945 Niedergampel, Kanton Wallis (CH); Bossard, Pierre, Dr., 1213 Onex, Kanton Genf (CH); Brieden, Walter, Dr., 3902 Brig-Glis, Kanton Wallis (CH); Duc, Laurent, Dr., 3971 Chermignon, Kanton Wallis (CH); Gordon, John, 3904 Naters, Kanton Wallis (CH); O'Murchu, Colm, Dr., 3930 Visp, Kanton Wallis (CH); Guggisberg, Yves, Dr., 3960 Sierre, Kanton Wallis (CH)
(74) Vertreter: Winter, Brandl, Fürniss, Hübner, Röss, Kaiser, Polte Partnerschaft

(56) Entgegenhaltungen:
- EP-A- 0 434 450
- EP-A- 0 590 685
- WO-A-93/17020
- WO-A-95/21161
- WO-A-97/45529
- US-A- 4 950 758
- VINCE, R. AND HUA, M.: "Synthesis and Anti-HIV Activity of Carbocyclic 2',3'-Didehydro-2',3'-dideoxy 2,6-Disubstituted Purine Nucleosides." J. MED. CHEM., Bd. 33, Nr. 1, 1990, Seiten 17-21, XP002109188
- TAYLOR, S.J.C. ET AL.: "Development of the Biocatalytic Resolution of 2-azabicyclo[2.2.1]hept-5-en-3-one as an entry to Single-Enantiomer Carbocyclic Nucleosides." TETRAHEDRON: ASYMMETRY, Bd. 4, Nr. 6, 1993, Seiten 1117-1128, XP002041316
- HILDEBRAND, S. ET AL.: "A Short Synthesis of (-)-Carbovir." HELV. CHIM. ACTA, Bd. 77, Nr. 5, 1994, Seiten 1236-1240, XP002109189
- CAMPBELL J. A. ET AL.: "Chirospecific Synthesis of Precursors of Cyclopentane and Cyclopentene Carbocyclic Nuceosides by [3+3]-Coupling and Transannular Alkylation." J. ORG. CHEM., Bd. 60, 1995, Seiten 4602-4616, XP002041314
- CHEMICAL ABSTRACTS, vol. 107, no. 5, 3. August 1987 (1987-08-03) Columbus, Ohio, US; abstract no. 39244, ALLAN, ROBIN D. ET AL: ".gamma.-Aminobutyric acid. Synthesis of analogs of GABA. XV. Preparation and resolution of some potent cyclopentene and cyclopentane derivatives" XP002113019 & AUST. J. CHEM. (1986), 39(6), 855-64 ,

### SEQUENZPROTOKOLL

(1) ALLGEMEINE ANGABEN:
   (i) ANMELDER:
      (A) NAME: LONZA AG
      (B) STRASSE: Muenchensteinerstrasse 38
      (C) ORT: Basel
      (E) LAND: Schweiz
      (F) POSTLEITZAHL: 4002
   (ii) BEZEICHNUNG DER ERFINDUNG: Verfahren zur Herstellung von (1S,4R)-oder (1R,4S)-4-(2-amino-6-chlor-9H-purin-9-yl)-2-cyclopenten-1-methanol
   (iii) ANWAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 20 Aminosäuren
      (B) ART: Aminosäure
      (C) STRANGFORM: nicht bekannt
      (D) TOPOLOGIE: nicht bekannt
   (ii) ART DES MOLEKÜLS: Protein
   (iii) HYPOTHETISCH: NEIN
   (iv) ANTISENSE: NEIN
   (v) ART DES FRAGMENTS: N-Terminus
   (vi) URSPRÜNGLICHE HERKUNFT:
      (B) STAMM: Rhodococcus erythropolis
      (C) INDIVIDUUM/ISOLAT: Rhodococcus erythropolis CB101
   (xi) SEQUENZBESCHREIBUNG: SEQ ID NO: 1:
   Thr Glu Gln Asn Leu His Trp Leu Ser Ala Thr Glu Met Ala Ala Ser
   1 5 10 15
   Val Ala Ser Asn
   20

## Beschreibung

Die Erfindung betrifft ein neues Verfahren zur Herstellung von (1S,4R)- und/oder (1R,4S)-4-(2-Amino-6-chlor-9-H-purin-9-yl)-2-cyclopenten-1-methanol der Formeln 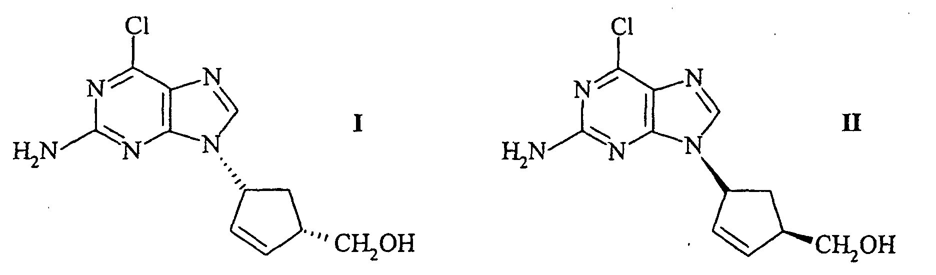 sowie ein neues Verfahren zur Herstellung von optisch aktiven Verbindungen der allgemeinen Formeln XVI und XVII 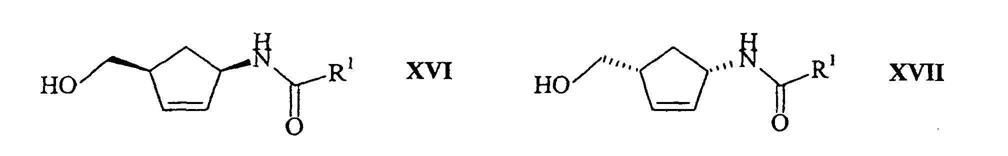 (1S,4R)-4-(2-Amino-6-chlor-9-H-purin-9-yl)-2-cyclopenten-1-methanol ist ein wichtiges Zwischenprodukt zur Herstellung von 2-Aminopurinnucleosiden wie z. B. zur Herstellung von (1S,4R)-4-[2-Amino-6-(cyclopropylamino)-9-H-purin-9-yl]-2-cyclopenten-1-methanol (WO-A-95 / 21 161) oder zur Herstellung von 1592U89 (J. Org. Chem., 1996, 61, 4192-4193; J. Org. Chem., 1996, 61, 7963-7966).

Die ältere WO-A-97/45529 beschreibt ein einstufiges Verfahren, bei dem aus racemischen N-substituierten 1-Amino-4-(hydroxymethyl)-2-cyclopentenderivaten mittels Racematspaltung durch Umsetzung mit spezifischen Mikroorganismen oder Enzymen (1S,4R)- oder (1R,4S)-1-Amino-4-(hydroxymethyl)-2-cyclopenten erhalten werden. Bei dieser Umsetzung fallen auch N-substituierte (1R,4S)- oder (1S,4R)-1-Amino-4-(hydroxymethyl)-2-cyclopentenderivate an.

R. Vince und M. Hua (J. Med. Chem. **33,** 17-21, 1990; US-A-4 950 758) beschreiben die Synthese von racemischem 4-(2-Amino-6-chlor-9-H-purin-9-yl)-2-cyclopenten-1-methanol ausgehend von diacetyliertem 1-Amino-4-hydroxymethyl-2-cyclopenten. Diese Verbindung wird in Gegenwart von Bariumhydroxid zu 1-Amino-4-(hydroxymethyl)-2-cyclopenten hydrolysiert, welches mit 2-Amino-4,6-dichlorpyrimidin zu racemischem cis-[4-[(2-Amino-6-chlor-4-pyrimidinyl)amino]-2-cyclopentenyl]carbinol reagiert. Letzteres wird in 5-Stellung durch Kupplung mit dem Diazoniumsalz aus *p*-Chloranilin und Natriumnitrit, gefolgt von einer Reduktion der Azogruppe in Gegenwart von Zink in Essigsäure unter Ausbildung von cis-[4-[(2,5-Diamino-6-chlor-4-pyrimidinyl)amino]-2-cyclopentenyl]carbinol aminiert. Formylierung der 5-Aminogruppe mittels Triethylorthoformat und anschließende Cyclisierung zum Purinring liefert das Endprodukt.

S.J.C. Taylor et al. (Tetrahedron. Asymmetry 4, 1117-1128, 1993) beschreiben die Synthese von optisch aktivem (1R,4S)-1-Amino-4-(hydroxymethyl)-2-cyclopenten ausgehend von (1R,4S)-2-Azabicyclo[2.2.1]hept-5-en-3-on. Bei dieser Synthese wird zunächst der Stickstoff des (1R,4S)-2-Azabicyclo[2.2.1]hept-5-en-3-ons mit einer tert-Butoxycarbonylgruppe aktiviert, anschliessend erfolgt die reduktive Lactamöffnung mittels Natriumcarbonat und schliesslich die Abspaltung der tert-Butoxycarbonylgruppe. (1R,4S)-2-Azabicyclo[2.2.1]hept-5-en-3-on wird durch enzymatische Racematspaltung aus racemischem 2-Azabicyclo[2.2.1]hept-5-en-3-on erhalten.

Ein Verfahren zur Herstellung von (1S,4R)-4-(2-Amino-6-chlor-9-H-purin-9-yl)-2-cyclopenten-1-methanol ausgehend von (1S,4R)-4-Amino-2-cyclopenten-1-methanol wird in der WO-A-95/21161 beschrieben. Nachteilig bei diesem Verfahren ist, dass das Edukt, das (1S,4R)-4-Amino-2-cyclopenten-1-methanol, ausschliesslich über das mit der kostspieligen BOC-Schutzgruppe (tert-Butyloxycarbonyl-Schutzgruppe) substituierte (±)-2-Azabicyclo-[2.2.1]hept-5-en-3-on erhalten werden kann (J. Org. Chem., 1995, 60, 4602-4616).

Aufgabe der Erfindung war es, ein einfaches, günstiges und wirtschaftlicheres Verfahren zur Herstellung von (1S,4R)- und/oder (1R,4S)-4-(2-Amino-6-chlor-9-H-purin-9-yl)-2-cyclopenten-1-methanol zur Verfügung zu stellen.

Diese Aufgabe wurde mit dem Verfahren gemäss Anspruch 1 gelöst.

Die erste Stufe des erfindungsgemässen Verfahrens wird derart durchgeführt, dass man (±)-2-Azabicyclo[2.2.1]hept-5-en-3-on der Formel 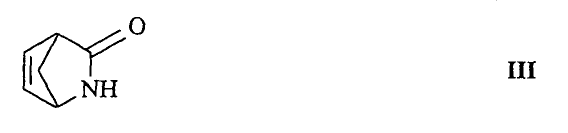 zu einem (±)-2-Azabicyclo[2.2.1]hept-5-en-3-on-derivat der allgemeinen Formel 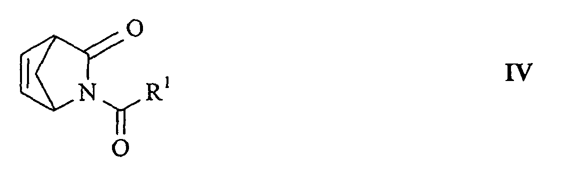 worin R¹ C₁-₄-Alkyl, C₁-₄-Alkoxy, Aryl oder Aryloxy bedeutet, acyliert.

Unter C₁-₄-Alkyl wird sowohl mit Halogen substituiertes als auch unsubstituiertes C₁-₄-Alkyl verstanden. Als Halogenatom kann F, Cl, Br oder J angewendet werden. Beispiele für C₁-₄-Alkyl sind Methyl, Ethyl, Propyl, Butyl, Isobutyl, tert-Butyl, Isopropyl, Chlormethyl, Brommethyl, Dichlormethyl, Dibrommethyl. Vorzugsweise wird als C₁-₄-Alkyl Methyl, Ethyl, Propyl, Butyl, Isobutyl oder Chlormethyl verwendet.

Als C₁-₄-Alkoxy kann beispielsweise Methoxy, Ethoxy, Propoxy, Butoxy, tert-Butoxy oder Isobutoxy verwendet werden. Vorzugsweise wird als C₁-₄-Alkoxy tert-Butoxy verwendet.

Als Aryl kann beispielsweise Phenyl oder Benzyl, vorzugsweise Phenyl, verwendet werden. Als Aryloxy kann beispielsweise Benzyloxy oder Phenoxy verwendet werden.

Das Edukt (±)-2-Azabicyclo[2.2.1]hept-5-en-3-on kann gemäss der EP-A-0 508 352 hergestellt werden.

Die Acylierung kann mit einem Carbonsäurehalogenid der allgemeinen Formel 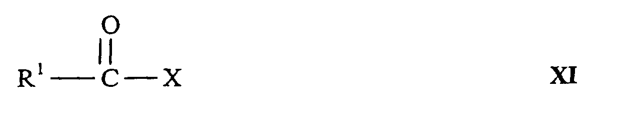 oder mit einem Carbonsäureanhydrid der allgemeinen Formel 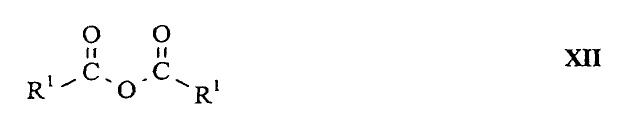 worin R¹ die genannte Bedeutung hat und X ein Halogenatom bedeutet, durchgeführt werden. Als Halogenatom X können F, Cl, Br oder J verwendet werden. Vorzugsweise wird Cl oder F verwendet.

Beispiele für Carbonsäurehalogenide sind: Acetylchlorid, Chloracetylchlorid, Buttersäurechlorid, Isobuttersäurechlorid, Phenylessigsäurechlorid, Chlorameisensäurebenzylester (Cbz-Cl), Propionsäurechlorid, Benzoylchlorid, Chlorameisensäureallylester oder tert-Butyloxycarbonylfluorid. Beispiele für Carbonsäureanhydride sind: tert-Butoxycarbonylanhydrid, Buttersäureanhydrid, Essigsäureanhydrid oder Propionsäureanhydrid.

Die Acylierung kann ohne Lösungsmittel oder mit einem aprotischen Lösungsmittel durchgeführt werden.

Zweckmässig wird die Acylierung in einem aprotischen Lösungsmittel durchgeführt. Als aprotisches Lösungsmittel sind beispielsweise Diisopropylether, Pyridin, Acetonitril, Dimethylformamid, Triethylamin, Tetrahydrofuran, Toluol, Methylenchlorid, N-methylpyrrolidon bzw. Mischungen von diesen geeignet.

Zweckmässig wird die Acylierung bei einer Temperatur von -80 bis 50 °C, vorzugsweise von 0 bis 25 °C, durchgeführt.

In der zweiten Stufe des erfindungsgemässen Verfahrens wird das (±)-2-Azabicyclo-[2.2. 1]hept-5-en-3-on-derivat der Formel IV zu einem Cyclopentenderivat der allgemeinen Formel 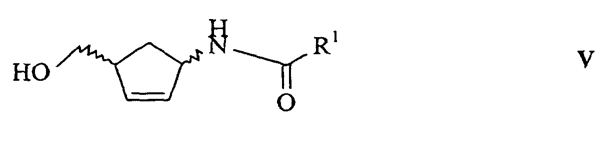 worin R¹ die genannte Bedeutung hat, reduziert.

Die Reduktion wird zweckmässig mit einem Alkalimetall- oder Erdalkalimetallborhydrid, mit einem Alkalimetall- oder Erdalkalimetallaluminiumhydrid oder mit Vitrid (Natrium-bis-(2-methoxyethoxy)aluminiumhydrid) durchgeführt. Als Alkalimetallaluminiumhydrid kann Natrium- oder Kaliumaluminiumhydrid verwendet werden. Als Alkalimetallborhydrid kann Natrium- oder Kaliumborhydrid verwendet werden. Als Erdalkalimetallborhydrid kann Calciumborhydrid verwendet werden.

Zweckmässig wird die Reduktion in einem protischen Lösungsmittel durchgeführt. Als protisches Lösungsmittel können niedere aliphatische Alkohole wie Methanol, Ethanol, Propanol, Isopropanol, Butanol, sec. Butanol, tert-Butanol, iso-Butanol oder Wasser bzw. eine Mischung von den genannten Alkoholen und Wasser verwendet werden.

Die Reduktion wird zweckmässig bei einer Temperatur von -40 bis 40 °C, vorzugsweise von 0 bis 20 °C, durchgeführt.

Die dritte Stufe des erfindungsgemässen Verfahrens, die Umsetzung des Cyclopentenderivats der allgemeinen Formel V zum (1R,4S)- und (1S,4R)-1-Amino-4-(hydroxymethyl)-2-cyclopenten der Formeln 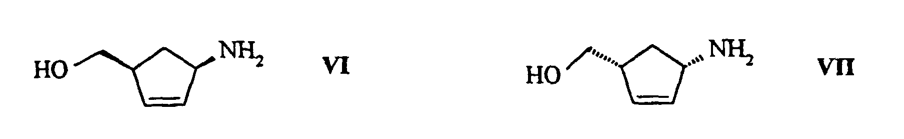 wird entweder mittels Mikroorganismen, die befähigt sind, ein Cyclopentenderivat der allgemeinen Formel V als einzige Stickstoff-, als einzige Kohlenstoff- oder als einzige Kohlenstoff- und Stickstoffquelle zu verwerten, Enzymextrakten daraus oder Enzymen mit N-Acetylaminoalkoholhydrolase-Aktivität oder mittels Penicillin G Acylasen durchgeführt. Bei dieser Biotransformation werden die acylierten (1S,4R)- und (1R,4S)-Aminoalkoholderivate umgesetzt, wobei die (1S,4R)- und (1R,4S)-1-Amino-4-(hydroxymethyl)-2-cyclopentene der Formeln VI und VII anfallen.

Für die Biotransformation sind alle Mikroorganismen geeignet, die ein Cyclopentenderivat der allgemeinen Formel V als einzige Stickstoffquelle, als einzige Kohlenstoffquelle oder als einzige Kohlenstoff- und Stickstoffquelle verwerten können. Solche Mikroorganismen und ihre Isolierung, daraus erhältliche Enzymextrakte und Enzyme mit N-Acetylaminoalkoholhydrolase-Aktivität sind in der älteren WO-A-97/45529 beschrieben. Insbesondere geeignet sind Mikroorganismen, die wenigstens das (1R,4S)-Enantiomere dieser Cyclopentenderivate V als einzige Stickstoffquelle, als einzige Kohlenstoffquelle oder als einzige Kohlenstoff- und Stickstoffquelle verwerten können.

Diese Mikroorganismen können insbesondere aus Bodenproben, Schlamm oder Abwasser unter Zuhilfenahme üblicher mikrobiologischer Techniken isoliert werden. Die Isolation der Mikroorganismen erfolgt derart, dass man diese in einem Nährmedium enthaltend ein Cyclopentenderivat der allgemeinen Formel 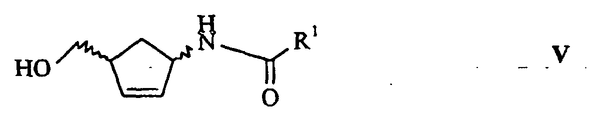 worin R¹ die genannte Bedeutung hat,
- als einzige Kohlenstoff- und Stickstoffquelle
- als einzige Stickstoffquelle mit einer geeigneten Kohlenstoffquelle oder
- als einzige Kohlenstoffquelle mit einer geeigneten Stickstoffquelle, auf übliche Weise züchtet.

Insbesondere können hierbei Mikroorganismen isoliert werden, die wenigstens das (1R,4S)-Enantiomere des Cyclopentens der Formel V als einzige Stickstoffquelle, als einzige Kohlenstoffquelle oder als einzige Kohlenstoff- und Stickstoffquelle verwerten können.

Als Cyclopentenderivate der allgemeinen Formel V sind beispielsweise geeignet: N-Acetyl-, N-Propionyl-, N-Isobutyryl-, N-tert-Butoxycarbonyl- (N-BOC), N-Butyryl- oder N-Phenylacetyl-1-amino-4-hydroxymethyl-2-cyclopenten.

Als geeignete Stickstoffquelle können die Mikroorganismen beispielsweise Ammonium, Nitrate, Aminosäuren oder Harnstoffe als Wachstumssubstrat nützen.

Als geeignete Kohlenstoffquelle können die Mikroorganismen beispielsweise Zucker, Zuckeralkohole, C₂-C₄-Carbonsäuren oder Aminosäuren als Wachstumssubstrat nützen. Als Zucker können Hexosen wie Glucose oder Pentosen verwendet werden. Als Zuckeralkohol kann beispielsweise Glycerin Verwendung finden. Als C₂-C₄-Carbonsäuren können beispielsweise Essigsäure oder Propionsäure verwendet werden. Als Aminosäuren können beispielsweise Leucin, Alanin oder Asparagin verwendet werden.

Als Selektions- und Anzuchtmedium können die in der Fachwelt üblichen verwendet werden. Geeignet ist beispielsweise das in Tabelle 1 beschriebene Medium oder ein Vollmedium (Medium enthaltend Hefe-Extrakt) wie z. B. "Nutrient Yeast Broth" (NYB): Vorzugsweise wird das in Tabelle 1 beschriebene Medium verwendet.

Während der Anzucht und Selektion werden zweckmässig die wirksamen Enzyme der Mikroorganismen induziert. Als Enzyminduktor können die Cyclopentenderivate der allgemeinen Formel V verwendet werden.

Üblicherweise erfolgt die Anzucht und Selektion bei einer Temperatur von 20 °C bis 40 °C, vorzugsweise von 30 °C bis 38 °C und bei einem pH zwischen pH 5,5 und pH 8, vorzugsweise zwischen pH 6,8 und pH 7,8.

Es wurde gefunden, daß die obigen Mikroorganismen, Enzymextrakte und Enzyme in der Lage sind, racemisches Cyclopentenderivat der Formel V wenigstens teilweise zu 1-Amino-4-(hydroxymethyl)-2-cyclopentenen der Formeln VI und VII umzusetzen. Dabei wird das (1R,4S)-Enantiomere des 1-Amino-4-(hydroxymethyl)-2-cyclopentens von den Mikroorganismen üblicherweise sehr viel schneller gebildet als das (1S,4R)-Enantiomere. Anscheinend verwerten die Mikroorganismen in einem Gemisch von (1R,4S)- und (1S,4R)-Enantiomeren von Cyclopentenderivat V bevorzugt das (1R,4S)-Enantiomere als Kohlenstoffund/oder Stickstoffquelle und hydrolysieren dieses deshalb bei der Biotransformation sehr viel rascher.

Vorzugsweise wird die Biotransformation mittels Mikroorganismen der Gattung Alcaligenes / Bordetella, Rhodococcus, Arthrobacter, Alcaligenes, Agrobacterium / Rhizobium, Bacillus, Pseudomonas oder Gordona, insbesondere der Spezies Alcaligenes / Bordetella FB 188 (DSM 11172), Rhodococcus erythropolis CB 101 (DSM 10686), Arthrobacter sp. HSZ5 (DSM 10328), Rhodococcus sp. FB 387 (DSM 11291), Alcaligenes xylosoxydans ssp. denitrificans HSZ17 (DSM10329), Agrobacterium / Rhizobium HSZ30, Bacillus simplex K2, Pseudomonas putida K32 oder Gordona sp. CB 100 (DSM 10687), sowie mit deren funktionell äquivalenten Varianten und Mutanten durchgeführt. Die Mikroorganismen DSM 10686 und 10687 wurden am 20.05.1996, die Mikroorganismen DSM 10328 und DSM 10329 am 06.11.1995, der Mikroorganismus DSM 11291 am 08.10.1996 und der Mikroorganismus DSM 11172 am 20.09.1996 bei der Deutschen Sammlung von Mikroorganismen und Zellkulturen GmbH, Mascheroderweg 1b, D38124 Braunschweig, gemäss Budapester Vertrag hinterlegt.

Unter "funktionell äquivalenten Varianten und Mutanten" werden Mikroorganismen verstanden, die sich von den oben genannten Mikroorganismen ableiten und im wesentlichen dieselben Eigenschaften und Funktionen wie die Ursprungsmikrooganismen besitzen. Derartige Varianten und Mutanten können zufällig, z. B. durch UV-Bestrahlung, gebildet werden.

Die Biotransformation kann nach üblichem Anzüchten dieser Mikroorganismen mit ruhenden Zellen (nicht wachsende Zellen, die keine Kohlenstoff- und Energiequelle mehr benötigen) oder mit wachsenden Zellen durchgeführt werden. Vorzugsweise wird die Biotransformation mit ruhenden Zellen durchgeführt.

Die für die Biotransformation geeigneten Enzymextrakte oder Enzyme mit N-Acetylaminoalkoholhydrolase-Aktivität können z. B. durch fachmännisch übliches Aufschliessen der beschriebenen Mikroorganismenzellen isoliert werden und besitzen dann hinsichtlich der Umsetzung der Cyclopentenderivate V entsprechende Selektivität. Hierzu kann beispielsweise die Ultraschall- oder French-Press-Methode verwendet werden. Bevorzugt werden diese Enzyme oder Enzymextrakte aus den Mikroorganismen Rhodococcus erythropolis CB101 (DSM 10686) isoliert.

Geeignete Penicillin G Acylasen lassen sich aus vielen Mikroorganismen, wie z. B. Bakterien oder Actinomyceten, im Speziellen aus den folgenden Mikroorganismen gewinnen: Escherichia coli ATCC 9637, Bacillus megaterium, Streptomyces lavendulae ATCC 13664, Nocardia sp. ATCC 13635, Providencia rettgeri ATCC 9918, Arthrobacter viscosus ATCC 15294, Rhodococcus fascians ATCC 12975, Streptomyces phaeochromogenes ATCC 21289, Achromobacter ATCC 23584 und Micrococcus roseus ATCC 416. Insbesondere werden käufliche Penicillin G Acylasen verwendet, beispielsweise Penicillin G Acylase EC 3.5.1.11 aus E. coli (Boehringer Mannheim) oder aus Bacillus megaterium. Penicillin G Acylasen hydrolysieren bevorzugt das (1R,4S)-Enantiomere der Cyclopentenderivate V. Bevorzugt werden immobilisierte Penicillin G Acylasen verwendet.

Die Biotransformation kann in fachmännisch üblichen Medien durchgeführt werden, wie beispielsweise in niedermolaren Phosphat-, Citrat- oder Hepes-Puffer, in Wasser, Vollmedien wie z. B. "Nutrient Yeast Broth" (NYB) oder in dem in Tabelle 1 beschriebenen. Vorzugsweise wird die Biotransformation in dem Medium gemäss Tabelle 1 oder in niedermolarem Phosphatpuffer durchgeführt.

Zweckmässig wird die Biotransformation unter einmaliger oder kontinuierlicher Zugabe des Cyclopentenderivates (Formel V) so durchgeführt, dass die Konzentration 10 Gew.%, vorzugsweise 2 Gew.% nicht übersteigt.

Der pH kann während der Biotransformation in einem Bereich von 5 bis 9, vorzugsweise von 6 bis 8 liegen. Zweckmässig wird die Biotransformation bei einer Temperatur von 20 bis 40 °C, vorzugsweise von 25 bis 30 °C, durchgeführt.

Der Verlauf der Umsetzung läßt sich beispielsweise mittels HPLC verfolgen. Zum Erhalt vornehmlich des (1R,4S)-Enantiomeren von 1-Amino-4-(hydroxymethyl)-2-cyclopenten wird die Umsetzung dann zweckmäßig bei Erreichen der optimalen oder gewünschten Enatiomerenreinheit abgebrochen, beispielsweise durch Zugabe von Säure. Die Umsetzung kann aber auch solange durchgeführt werden, bis sich daneben ausreichend (1S,4R)-Enantiomer gebildet hat.

| **Taxonomische Beschreibung von Alcaligenes / Bordetella FB 188 (DSM 11172)** | |
|---|---|
| Zellform | Stäbchen |
| Breite µm | 0,5 - 0,6 |
| Länge µm | 1,0 - 2,5 |
| Beweglichkeit | + |
| Begeisselung | peritrich |
| Gram-Reaktion | - |
| Lyse durch 3% KOH | + |
| Aminopeptidase (Cerny) | + |
| Sporen | - |
| Oxidase | + |
| Catalase | + |
| ADH (Alkoholdehydrogenase) | - |
| NO₂ aus NO₃ | - |
| Denitrifikation | - |
| Urease | - |
| Hydrolyse von Gelatine | - |

| Säure aus (OF-Test): | |
|---|---|
| Glucose | - |
| Fructose | - |
| Arabinose | - |
| Adipat | + |
| Caprat | + |
| Citrat | + |
| Malat | + |
| Mannitol | - |

### Taxonomische Beschreibung von Rhodococcus erythropolis CB 101 (DSM 106 86)

. Morphologie und Farbe der Kolonien: Kurze verzweigte Hyphen, die im Alter in Stäbchen und Kokken zerfallen, Kolonien glänzend zum Teil zerfliessend, beige mit rosa Ton, RAL 1001;
. Diagnostizierte Aminosäure des Peptidoglycans: meso-Diaminopimelinsäure;
. Mycolsäuren: Rhodococcus-Mycolsäuren; Die Bestimmung der Mycolsäure-Kettenlänge (C₃₂ - C₄₄) und der Vergleich der Daten mit den Einträgen in der DSM-Mycolsäuredatenbank ergab eine sehr hohe Ähnlichkeit zu den Mustern der Rhodococcus erythropolis Stämmen (Similarität, 0,588).
. Fettsäuremuster: Unverzweigte, gesättigte und ungesättigte Fettsäuren plus Tuberculostearinsäure.
. Bei der Partial-Sequenzierung der 16S rDNA des Stammes wurde eine hohe Übereinstimmung (100%) mit den Sequenzen der spezifischen Regionen von Rhodococcus erythropolis gefunden.

Das Identifizierungsergebnis ist eindeutig, da drei voneinander unabhängige Methoden (Mycolsäuren, Fettsäuren, 16S rDNA) des Stammes der Spezies Rhodococcus erythropolis zugeordnet haben.

### Taxonomische Beschreibung von Gordona sp. CB 100 (DSM 10687)

. Morphologie und Farbe der Kolonien: Kurze verzweigte Hyphen, die im Alter in Stäbchen und Kokken zerfallen, Kolonien hellorange, (RAL 2008);
. Diagnostizierte Aminosäure des Peptidoglycans: meso-Diaminopimelinsäure;
. Menachinonmuster: MK-9 (H₂) 100%;
. Mycolsäuren: Gordona-Mycolsäuren; Die Bestimmung der Mycolsäure-Kettenlänge (C₅₀-C₆₀) erfolgte mit Hilfe von Hochtemperatur-Gaschromatographie. Dieses Muster entspricht dem Muster, wie es bei Vertretern der Gattung Gordona gefunden wird.
. Fettsäuremuster: Unverzweigte, gesättigte und ungesättigte Fettsäuren plus Tuberculostearinsäure.
. Bei der Partial-Sequenzierung der 165 rDNA des Stammes konnte nur eine relativ niedrige Übereinstimmung 98,8% mit den Sequenzen der spezifischen Regionen von Gordona rubropertincta gefunden werden.

Aufgrund der vorliegenden Ergebnisse (Menachinone, Mycolsäuren, Fettsäuren 16S rDNA) kann das Isolat zwar eindeutig der Gattung Gordona zugeordnet werden, eine Zuordnung zu einer bekannten Gordona Spezies ist auf Grund dieser Ergebnisse nicht möglich. Es ist daher anzunehmen, dass es sich bei dem Stamm DSM 10687 um eine neue bisher nicht beschriebene Spezies der Gattung Gordona handelt.

| **Taxonomische Beschreibung von Alcaligenes xylosoxydans ssp. denitrificans HSZ 17 (DSM 10329)** | |
|---|---|
| Eigenschaften des Stammes | |
| Zellform | Stäbchen |
| Breite µm | 0,5 - 0,6 |
| Länge µm | 1,5 - 3,0 |
| | |
| Beweglichkeit | + |
| | |
| Begeisselung | peritrich |
| | |
| Gram-Reaktion | - |
| Lyse durch 3% KOH | + |
| Aminopeptidase (Cerny) | + |
| | |
| Sporen | - |
| | |
| Oxidase | + |
| | |
| Catalase | + |
| | |
| Wachstum anaerob | - |
| | |
| ADH (Alkoholdehydrogenase) | + |
| | |
| NO₂ aus NO₃ | + |
| | |
| Denitrifikation | + |
| | |
| Urease | - |
| | |

| Hydrolyse von | |
|---|---|
| Gelatine | - |
| Tween 80 | - |
| | |

| Säure aus (OF-Test): | |
|---|---|
| Glucose aerob | - |
| Xylose 80 | - |
| | |

| Substratverwertung | |
|---|---|
| Glucose | - |
| Fructose | - |
| Arabinose | - |
| Citrat | + |
| Malat | + |
| Mannitol | - |

| **Taxonomische Beschreibung von Arthrobacter sp. HSZ5 (DSM 10328))** | |
|---|---|
| Charakterisierung: | Gram-positive unregelmässige Stäbchen mit einem ausgeprägten Stäbchen-Kokken Wachstumszyklus; strikt aerob; keine Säure- oder Gasbildung aus Glucose. |
| | |
| Beweglichkeit | - |
| Sporen | - |
| Katalase | + |
| | |
| *meso*-Diaminopimelinsäure in der Zellwand: | nein |
| Peptidoglycan-Typ: | A3α, L-Lys-L-Ser-L-Thr-L-Ala |
| 16S rDNA Sequenz-Ähnlichkeit: | Sequenzierung des Bereichs mit der grössten Variabilität ergab als höchste Werte 98,2% mit Arthrobacter pascens, A. ramosus und A. oxydans |

| **Taxonomische Beschreibung von Agrobacterium/Rhizobium HSZ30** | |
|---|---|
| Zellform | pleomorphe Stäbchen |
| Breite [µm] | 0,6 - 1,0 |
| Länge [µm] | 1,5 - 3,0 |
| | |
| Gram-Reaktion | - |
| Lyse durch 3% KOH | + |
| Aminopeptidase | + |
| | |
| Sporen | - |
| | |
| Oxidase | + |
| | |
| Catalase | + |
| | |
| Beweglichkeit | + |
| | |
| Wachstum anaerob | - |
| | |
| Nitrit aus Nitrat | - |
| | |
| Denitrifikation | - |
| | |
| Urease | + |
| | |
| Hydrolyse von Gelatine | - |

| Säure aus: | |
|---|---|
| L-Arabinose | + |
| Galactose | - |
| Melezitose | - |
| Fucose | + |
| Arabitol | - |
| Mannitol | - |
| Erythritol | - |
| | |
| Alkalisierung von Lackmusmilch | + |
| | |
| Ketolactose | - |

Die partielle Sequenzierung der 16S rDNA ergab vergleichbar hohe Ähnlichkeiten von ca. 96% zu Vertretern der Gattungen Agrobacterium und Rhizobium. Eine eindeutige Zuordnung zu einer innerhalb dieser Gattungen beschriebenen Art ist nicht möglich.

| **Taxonomische Beschreibung von Bacillus simplex K2** | |
|---|---|
| Zellform | Stäbchen |
| Breite [µm] | 0,8 - 1,0 |
| Länge [µm] | 3,0 - 5,0 |
| | |
| Sporen | - |
| ellipsoid | - |
| rund | - |
| Sporangium | - |
| | |
| Catalase | + |
| | |
| Anaerobes Wachstum | - |
| | |
| VP Reaktion | k. W. |
| | |
| | |
| Maximale Temperatur | |
| Wachstum positiv bei °C | 40 |
| Wachstum negativ bei °C | 45 |
| | |
| Wachstum in | |
| Medium pH 5,7 | - |
| NaCl 2% | + |
| 5% | - |
| 7% | - |
| 10% | - |
| Lysozym-Medium | + |
| Säure aus (ASS) | |
| D-Glucose | + |
| L-Arabinose | + |
| D-Xylose | - |
| D-Mannit | + |
| D-Fructose | + |
| | |
| Gas aus Fructose | - |
| | |
| Lecithinase | - |
| | |
| Hydrolyse von | |
| Stärke | + |
| Gelatine | + |
| Casein | - |
| Tween 80 | + |
| Äskulin | - |
| | |
| Verwertung von | |
| Citrat | + |
| Propionat | - |
| | |
| Nitrit aus Nitrat | + |
| | |
| Indol | - |
| | |
| Phenylalanindesaminase | - |
| | |
| Arginindihydrolase | - |

Die Analyse der zellulären Fettsäuren ergab eine Bestätigung der Zuordnung zur Gattung Bacillus.

Die partielle Sequenzierung der 16S rDNA ergab eine Ähnlichkeit von 100% zu Bacillus simplex.

| **Taxonomische Beschreibung von Pseudomonas putida K32K2** | |
|---|---|
| Zellform | Stäbchen |
| Breite [µm] | 0,8 - 0,9 |
| Länge [µm] | 1,5 - 4,0 |
| | |
| Beweglichkeit | + |
| Begeisselung | polar>1 |
| | |
| Gram-Reaktion | - |
| Lyse durch 3% KOH | + |
| Aminopeptidase | + |
| | |
| Sporen | - |
| | |
| Oxidase | + |
| | |
| Catalase | + |
| | |
| Wachstum anaerob | - |
| | |

| Pigmente | |
|---|---|
| fluoreszierend | + |
| Pyocyanin | - |
| | |
| ADH | + |
| | |
| Nitrit aus Nitrat | - |
| | |
| Denitrifikation | - |
| | |
| Urease | - |
| | |
| Hydrolyse von Gelatine | - |
| | |

| Substratverwertung | |
|---|---|
| Adipat | - |
| Citrat | + |
| Malat | + |
| D-Mandelat | + |
| Phenylacetat | + |
| D-Tartrat | - |
| D-Glucose | + |
| Trehalose | - |
| Mannitol | - |
| Benzoylformiat | - |
| Propylenglycol | + |
| Butylamin | + |
| Benzylamin | + |
| Tryptamin | - |
| Acetamid | + |
| Hippurat | + |

Das Profil der zellulären Fettsäuren ist typisch für Pseudomonas putida.

Das Profil der zellulären Fettsäuren ist typisch für Pseudomonas putida. Die partielle Sequenzierung der 16S rDNA ergab Ähnlichkeiten von ca. 98% zu Pseudomonas mendocina und Pseudomonas alcaligenes. Die Ähnlichkeit zu Pseudomonas putida betrug 97,4%.

### Taxonomische Beschreibung von Rhodococcus sp. FB 387 (DSM 11291)

1. Morphologie und Farbe der Kolonien: Kurze verzweigte Hyphen, die im Alter in Stäbchen und Kokken zerfalllen, Kolonien stumpf, hell rot-orange RAL 2008;
2. Diagnostizierte Aminosäure des Peptidoglycans: meso-Diaminopimelinsäure;
3. Mycolsäuren: Rhodococcus-Mycolsäuren;
   Die Bestimmung der Mycolsäure-Kettenlänge (C₃₂-C₄₄) und der Vergleich der Daten mit den Einträgen in der DSMZ-Mycolsäuredatenbank ergab nur eine sehr geringe Ähnlichkeit zu den Mustern von Rhodococcus ruber Stämmen (Similarität, 0,019). Dieser Korrelationsfaktor ist zu gering, um zur Spezies Identifizierung herangezogen werden zu können.
4. Fettsäuremuster: Unverzweigte, gesättigte und ungesättigte Fettsäuren plus Tuberculostearinsäure.
   Dieses Fettsäuremuster ist diagnostisch für alle Vertreter der Gattung Rhodococcus und ihre nahen Verwandten wie Mycobacterium, Nocardia und Gordona. Es wurde versucht, unter Einbeziehung der qualitativen und quantitativen Unterschiede im Fettsäuremuster eine Differenzierung zur Spezies-Ebene durchzuführen. Mit Hilfe von numerischen Methoden wurde das Fettsäure-Muster von Rhodococcus sp. FB 387 mit den Einträgen der Datenbank verglichen. Auch mit dieser Methode konnte Rhodococcus sp. FB 387 auf Grund der geringen Similarität (0,063) keiner beschriebenen Rhodococcus-Spezies zugeordnet werden.
5. Bei der Partial-Sequenzierung der 16S rDNA des Stammes wurde 96-818 mit einer Korrelation von 97,9% Rhodococcus opacus zugeordnet. Diese Sequenzübereinstimmung liegt weit unter der von 99,5% wie sie für eine eindeutige Spezies-Zuordnung in diesem Taxon verlangt wird.

Auf Grund der vorliegenden Ergebnisse kann man davon ausgehen, dass es sich bei dem Stamm Rhodococcus sp. FB 387 um eine neue, bisher nicht beschriebene Rhodococcus-Spezies handelt.

In der vierten Stufe wird das Cyclopentenderivat der Formel VI oder VII, falls gewünscht nach Auftrennung der Isomeren, mit N-(2-Amino-4,6-dichlorpyrimidin-5-yl)formamid der Formel 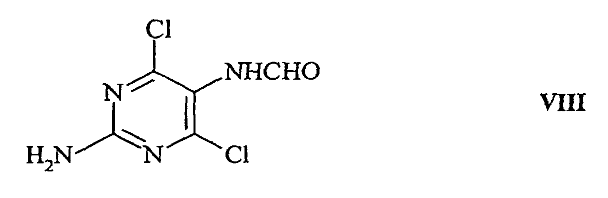 in das (1S,4R)- oder (1R,4S)-4-[(2-Amino-6-chlor-5-formamido-4-pyrimidinyl)-amino]-2-cyclopenten-1-methanol der Formel IX oder X 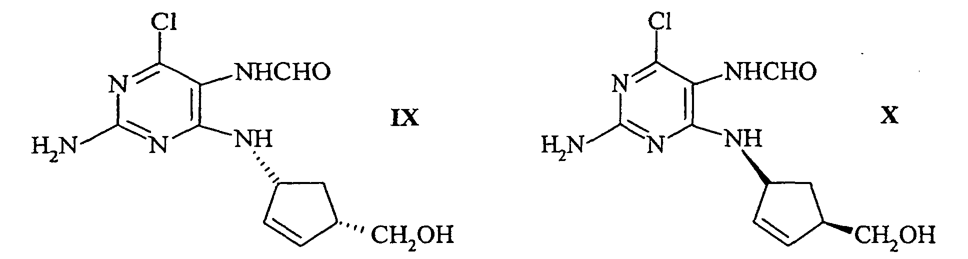 überführt.

Das N-(2-Amino-4,6-dichlorpyrimidin-5-yl)formamid kann gemäss der WO-A-95 / 21 161 hergestellt werden.

Zweckmässig wird die Umsetzung in der vierten Stufe in Gegenwart einer Base durchgeführt. Als Base können organische Basen oder anorganische Basen eingesetzt werden. Als organische Basen können Trialkylamine angewendet werden. Beispiele für Trialkylamine sind Triethylamin, Tributylamin, Tribenzylamin, Pyridin oder N-Methylpyrrolidin. Als anorganische Basen können beispielsweise Alkali- oder Erdalkalicarbonate bzw. Alkali- oder Erdalkalihydrogencarbonate, beispielsweise Kaliumcarbonat und Natriumhydrogencarbonat, eingesetzt werden.

Zweckmässig wird die Umsetzung in der vierten Stufe in einem protischen Lösungsmittel durchgeführt. Als protische Lösungsmittel können niedere aliphatische Alkohole wie Methanol, Ethanol, Propanol, Isopropanol, Butanol oder Isobutanol verwendet werden.

Die Umsetzung in der vierten Stufe wird zweckmässig bei einer Temperatur von 0 bis 150 °C, vorzugsweise von 20 bis 100 °C, durchgeführt.

In der fünften Stufe wird das (1S,4R)- oder (1R,4S)-4-[(2-Amino-6-chlor-5-formamido-4-pyrimidinyl)-amino]-2-cyclopenten-1-methanol (Formel IX, X) auf bekannte Weise gemäss der WO-A-95 / 21 161 zum Endprodukt gemäss Formel I oder II cyclisiert.

Üblicherweise wird die Cyclisierung gelöst in Trialkylorthoformaten in Gegenwart von einer konzentrierten wässrigen Säure durchgeführt. Als Trialkylorthoformate können beispielsweise Trimethyl- oder Triethylorthoformat Verwendung finden. Als wässrige Säure kann beispielsweise Chlorwasserstoffsäure, Schwefelsäure oder Methansulfonsäure verwendet werden.

Ein weiterer Bestandteil der Erfindung ist ein Verfahren zur Herstellung der optisch aktiven Verbindungen der allgemeinen Formeln 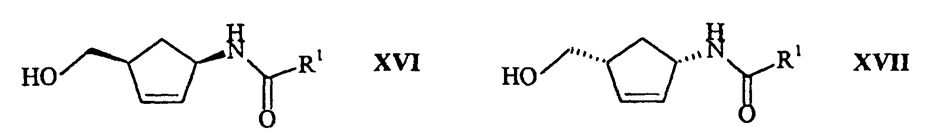 worin R¹ die genannte Bedeutung hat. Diese Verbindungen sind erhältlich durch Umsetzen eines Cyclopentenderivats der allgemeinen Formel 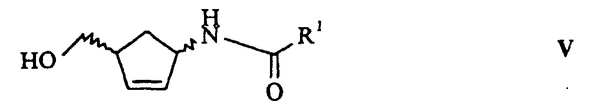 worin R¹ die genannte Bedeutung hat, mittels der bereits zuvor beschriebenen Mikroorganismen, Enzymextrakten, N-Acetylaminoalkoholhydrolasen oder Penicillin G Acylasen zu den (1R,4S)- und/oder (1S,4R)-1-Amino-4-(hydroxymethyl)-2-cyclopentenen gemäss den Formeln 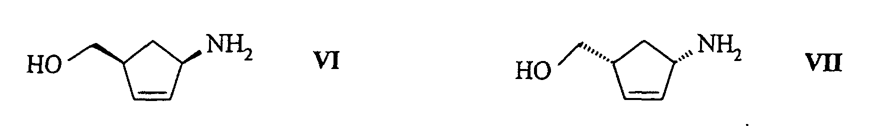 wobei letztere zu den Verbindungen der Formel XVI oder XVII acyliert werden.

Das Cyclopentenderivat der Formel V läßt sich beispielsweise wie oben beschrieben herstellen. Sowohl die mikrobiologische Umsetzung als auch die Acylierung werden unter an sich gleichen Bedingungen wie zuvor beschrieben durchgeführt.

Zweckmässig wird diese Acylierung bei einer Temperatur von 20 °C bis 100 °C, vorzugsweise von 0 °C bis 80 °C, durchgeführt.

Beispiele für verfahrensgemäß herstellbare optisch aktive Verbindungen sind:
(1R,4S)-N-tert-Butoxycarbonyl-1-amino-4-(hydroxymethyl)-2-cyclopenten mit einem ee-Wert von 98%,
(1R,4S)-N-Acetyl-1-amino-4-(hydroxymethyl)-2-cyclopenten mit einem ee-Wert von 98 %,
(1R,4S)-N-Butyryl-1-amino-4-(hydroxymethyl)-2-cyclopenten mit einem ee-Wert von 98 %,
optisch aktives (1S,4R)-N-Acetyl-1-amino-4-(hydroxymethyl)-2-cyclopenten und optisch aktives (1S,4R)-N-Butyryl-1-amino-4-(hydroxymethyl)-2-cyclopenten.

Von diesen Verbindungen ist das optisch aktive (1R,4S)-N-Butyryl-1-amino-4-(hydroxymethyl)-2-cyclopenten eine in der Literatur noch nicht beschriebene Verbindung. Demnach ist auch Bestandteil der Erfindung optisch aktives (1R,4S)-N-Butyryl-1-amino-4-(hydroxymethyl)-2-cyclopenten mit mehr als 0 % enantiomeren Überschuss, vorzugsweise in einem mindestens 80 %, 90 % oder 95 % enantiomeren Überschuss, insbesondere in einem mindestens 98 % enantiomeren Überschuss. Diese optisch aktiven Verbindungen können auf fachmännisch bekannte Weise in das in der Literatur noch nicht beschriebene racemische N-Butyryl-1-amino-4-(hydroxymethyl)-2-cyclopenten racemisiert werden.

Das Verfahren zur Herstellung von einem racemischen oder optisch aktiven 4-(Hydroxymethyl)-2-cyclopentenderivat der allgemeinen Formel 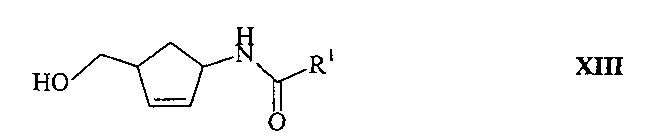 worin R¹ die genannte Bedeutung hat, wird derart durchgeführt, dass man in der ersten Stufe eine Cyclopenten-4-carbonsäure der Formel 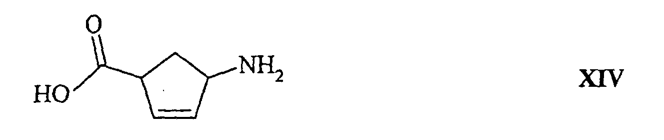 in Form des Racemats oder eines seiner optisch aktiven Isomere, mit einem Carbonsäurehalogenid der allgemeinen Formel 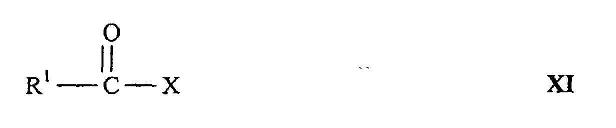 worin R¹ und X die genannte Bedeutung haben, in ein racemisches oder optisch aktives Cyclopenten-4-carbonsäurederivat der allgemeinen Formel 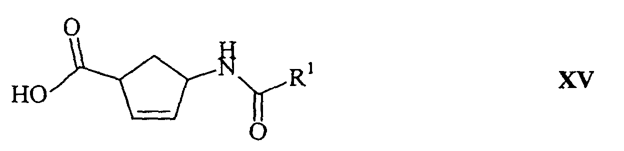 acyliert und dieses in der zweiten Stufe zum gewünschten Produkt gemäss Formel XIII reduziert.

Unter optisch aktiven 4-(Hydroxymethyl)-2-cyclopentenderivaten (Formel XIII) und optisch aktiven Cyclopenten-4-carbonsäurederivaten (Formel XV) werden die entsprechenden (1R,4S)-oder (1S,4R)-Isomere verstanden.

Die erste Stufe dieses Verfahrens, die Acylierung, wird mit einem Carbonsäurehalogenid der allgemeinen Formel XI durchgeführt. Als Carbonsäurehalogenide können die gleichen wie die zuvor beschriebenen verwendet werden, vorzugsweise wird tert. Butyloxycarbonylfluorid verwendet.

Zweckmässig wird die Umsetzung in der ersten Stufe bei einem pH-Wert von 8 bis 14, vorzugsweise von 12 bis 14, und bei einer Temperatur von 0 bis 50 °C, vorzugsweise von 15 bis 25 °C, durchgeführt.

Als Lösungsmittel sind geeignet Mischungen von Wasser mit Ethern. Als Ether kann Dioxan, Tetrahydrofuran, Diethylether, Glycoldimethyl- oder -diethylether verwendet werden.

Die zweite Stufe des Verfahrens, die Reduktion, kann mit einem Alkalialuminiumhydrid, mit einem Boran-di-C₁₋₄-alkylsulfid-Komplex oder mit einem Boran-tetrahydrofuran-Komplex durchgeführt werden. Als Alkalialuminiumhydrid kann Lithium-, Natrium- oder Kaliumaluminiumhydrid verwendet werden, vorzugsweise wird Lithiumaluminiumhydrid verwendet. Als Boran-di-C₁₋₄-alkylsulfid-Komplex können Boran-dimethylsulfid-, Boran-diethylsulfid-, Boran-dipropylsulfid- oder Boran-dibutylsulfid-Komplexe verwendet werden. Vorzugsweise wird Boran-dimethylsulfid-Komplex verwendet.

Zweckmässig wird als Lösungsmittel in der zweiten Stufe einer der oben aufgeführten Ether, ohne Wasser, verwendet.

Die Umsetzung in der zweiten Stufe kann bei einer Temperatur von -50 bis 5 °C, vorzugsweise von -25 bis -10 °C durchgeführt werden.

### Beispiele:

### Beispiel 1

### Herstellung von (±)-2-Acetyl-2-azabicyclo[2.2.1]hept-5-en-3-on

100 g (±)-2-Azabicyclo[2.2.1]hept-5-en-3-on wurden unter Stickstoff in Acetonitril (800 ml) und Pyridin (161,26 ml) gelöst. Bei 12 °C wurden 104,5 g Acetylchlorid über 2 Stunden zugetropft. Das Gemisch wurde noch während 4,5 Stunden bei Raumtemperatur gerührt. Die Mischung wurde mit 800 ml Wasser versetzt und das Acetonitril wurde unter Vakuum eingedampft. Die Wasserphase wurde 3mal mit 400 ml Ethylacetat extrahiert. Die vereinigten org. Phasen wurden noch mit 1N HCl (400 ml), Wasser (400 ml), gesättigtem NaCl (400 ml) gewaschen, mit Magnesiumsulfat getrocknet und voll eingedampft. Der Rückstand wurde in Methylenchlorid aufgenommen und über Kieselgel filtriert. Das Filtrat wurde eingeengt und das Produkt wurde durch Destillation gereinigt. Man erhielt 107,76 g Produkt als klare Flüssigkeit. Die Ausbeute betrug 71%.
Kp_{0.07 torr} : 51°C
- ¹H-NMR (CDCl₃): δ [ppm]: 2,25 (AB syst., 2H);
- 400 MHz: 2,8 (s, 3H);
3,42 (m, 1H);
5,30 (m, 1H);
6,89 (m, 1H);
6,92 (m, 1H);

### Beispiel 2

### Herstellung von (±)-2-Butyryl-2-azabicyclo[2.2.1]hept-5-en-3-on

100,3 g (±)-2-Azabicyclo[2.2.1]hept-5-en-3-on wurden unter Stickstoff in Acetonitril (720 ml) und Pyridin (142 ml) gelöst. Bei 12 °C wurden 141,8 g Buttersäurechlorid über 1 Stunde zugetropft. Die Reaktion wurde noch während 3 Stunden bei Raumtemperatur gerührt. Das Gemisch wurde mit 720 ml Wasser versetzt und die Phasen wurden getrennt. Das Acetonitril wurde unter Vakuum eingedampft und die Wasserphase wurde 3mal mit Ethylacetat (300 ml) extrahiert. Die vereinigten org. Phasen wurden noch mit 1N HCl (350 ml), gesättigtem NaCl (400 ml) und Wasser (500 ml) gewaschen, mit Magnesiumsulfat getrocknet und voll eingedampft. Das Produkt wurde durch Destillation gereinigt. Man erhielt 107,76 g Produkt als klare Flüssigkeit. Die Ausbeute betrug 85%.
Kp_{0.05 torr} : 70 °C
- ¹H-NMR (CDCl₃) : δ [ppm]: 0,98 (t, J = 8.5 Hz, 3H);
- 400 MHz: 1,58 - 1,65 ( 2H);
2,23 (AB syst., 2H);
2,82 - 2,90 ( 2H);
3,42 (m, 1H);
5,30 (m, 1H);
6,62 (m, 1H);
6,90 (m, 1H).

### Beispiel 3

### Herstellung von (±)-2-Phenylacetyl-2-azabicyclo[2.2.1]hept-5-en-3-on

33,4 g (±)-2-Azabicyclo[2.2.1]hept-5-en-3-on wurden unter Stickstoff in Acetonitril (240 ml) und Pyridin (48,3 ml) gelöst. Bei 12 °C wurden 68,6 g Phenylessigsäurechlorid über 30 Minuten zugetropft. Die Mischung wurde noch während 3,5 Stunden bei Raumtemperatur gerührt. Das Gemisch wurde mit 240 ml Wasser versetzt. Das Acetonitril wurde unter Vakuum eingedampft und die Wasserphase wurde 3mal mit Ethylacetat (150 ml) extrahiert. Die vereinigten org. Phasen wurden noch mit 1N HCl (150 ml), gesättigtem NaCl (150 ml) und Wasser (150 ml) gewaschen, mit Magnesiumsulfat getrocknet und voll eingedampft. Das Rohprodukt wurde über Kieselgel abfiltriert (Hexan:Ethylacetat = 1:1). Man erhielt 78,34 g des rohen Produktes als gelbes Öl.

### Beispiel 4

### Herstellung von (±)-2-Propionyl-2-azabicyclo[2.2.1]hept-5-en-3-on

47 g (±)-2-Azabicyclo[2.2.1]hept-5-en-3-on wurden unter Stickstoff in Acetonitril (325 ml) und Pyridin (41 ml) gelöst. Bei 12 °C wurden 43,9 g Propionsäurechlorid über 1 h zugetropft. Die Reaktion wurde noch während 5 h bei Raumtemperatur gerührt. Das Gemisch wurde mit 145 ml Wasser versetzt und das Acetonitril unter Vakuum eingedampft. Die wässrige Phase wurde 3mal mit 115 ml Ethylacetat extrahiert. Die vereinigten organischen Phasen wurden noch mit 1N HCl (140 ml), gesättigtem NaHCO₃ (40 ml) und NaCl (40 ml) Lösungen gewaschen, mit Natriumsulfat getrocknet und vollständig eingedampft. Der Rückstand wurde durch Destillation gereinigt. Man erhielt 55,8 g Titelverbindung, die bei Stehenlassen erstarrt. Die Ausbeute betrug 81,6%.
Kp 2,8 mbar 75 - 80 °C
Smp.: 54 -56 °C
- ¹H-NMR (DMSO-d₆) : δ [ppm]: 0,95 (t, 3H);
- 400 MHz: 2,10 (quad., 1H); 2,28 (quad., 1H); 2,64 (m, 2H); 3,42 (s, 1H); 5,16 (s, 1H); 6,78 (m, 1H): 6,96 (m, 1H).

### Beispiel 5

### Herstellung von (±)-2-Isobutyryl-2-azabicyclo[2.2.1]hept-5-en-3-on

45,1 g (±)-2-Azabicyclo[2.2.1 ]hept-5-en-3-on wurden unter Stickstoff in Acetonitril (310 ml) und Pyridin (39 ml) gelöst. Bei 10 °C wurden 54,1 g Isobuttersäurechlorid über 1 h zugetropft. Die Reaktion wurde noch während 5 h bei Raumtemperatur gerührt. Das Gemisch wurde mit 140 ml Wasser versetzt und das Acetonitril unter Vakuum eingedampft. Die wässrige Phase wurde mit 4mal 120 ml Ethylacetat extrahiert. Die vereinigten organischen Phasen wurden noch mit 1N HCl (50 ml) gesättigten NaHCO₃ (50 ml) und NaCl (50 ml) Lösungen gewaschen, mit Natriumsulfat getrocknet und vollständig eingedampft. Der Rückstand wurde in n-Hexan (240 ml) mit Aktivkohle unter Rückfluss gekocht. Nach Filtration der Aktivkohle wurde das Filtrat bei 0 °C gekühlt und die Titelverbindung filtriert. Man erhielt 54,5 g Produkt. Die Ausbeute betrug 76%.
Smp.: 41 - 42 °C
- ¹H-NMR (DMSO-d₆) : δ [ppm]: 0,92 (d, 3H);
- 400 MHz: 1,06 (d, 3H); 2,10 (m, 1H); 2,28 (m, 1H); 3,40 (m, 2H); 5,16 (s, 1H); 6,78 (m, 1H); 7,92 (m, 1H).

### Beispiel 6

### Herstellung von (±)-2-Chloracetyl-2-azabicyclo[2.2.1]hept-5-en-3-on

10,1 g (±)-2-Azabicyclo[2.2.1]hept-5-en-3-on wurden unter Stickstoff und bei 10 °C in einer Mischung von Dichlormethan (10 ml), Pyridin (8,4 ml) und 0,22 g 4-N,N-dimethylaminopyridin gelöst. 13,5 g Chloracetylchlorid wurden über 1 h zugetropft. Die Temperatur stieg bis 44 °C. Das Gemisch wurde bei Raumtemperatur während 2 h weitergerührt. Die Lösung wurde mit 100 ml Wasser addiert. Nach Phasentrennung wurde die wässrige Phase mit 100 ml Dichlormethan extrahiert. Die vereinigten organischen Phasen wurden mit Natriumsulfat getrocknet und vollständig eingedampft. Der Rückstand wurde in 100 ml Diisopropylether unter Rückfluss in Gegenwart von 1 g Aktivkohle über 10 Minuten gekocht. Nach heisser Filtration wurde das Filtrat bis zur Raumtemperatur abgekühlt, der Feststoff filtriert und getrocknet. Man erhielt 10,35 g Titelverbindung. Die Ausbeute betrug 60%.
Smp.: 86 - 88 °C
- ¹H-NMR (CDCl₃) : δ [ppm]: 2,28 (d, 1H);
- 400 MHz: 2,40 (d, 1H); 3,48 (s, 1H); 4,56 (d, 2H); 5,30 (s, 1H); 6,70 (d, 1H); 6,94 (m, 1H).

### Beispiel 7

### Herstellung von (±)-N-Acetyl-1-amino-4-(hydroxymethyl)-2-cyclopenten

79,56 g (±)-2-Acetyl-2-azabicyclo[2.2.1]hept-5-en-3-on wurden unter Stickstoff in Ethanol (450 ml) gelöst und auf -10 °C gekühlt. 19,8 g Natriumborhydrid wurden portionsweise über 45 Minuten zugegeben. Die Reaktion wurde bei 0 °C noch 3 Stunden gerührt und dann wurde der pH auf 1,8 mit konz. Schwefelsäure eingestellt. Dieses Gemisch wurde mit Ethylacetat (200 ml) versetzt und die Feststoffe wurden abfiltriert. Dann wurde es voll eingedampft. Der Rückstand wurde in Wasser aufgenommen, mit Methylenchlorid gewaschen und eingedampft. Das Rohprodukt wurde noch mit einer Kieselgel Filtration gereinigt. Man erhielt 51,83 g Produkt als weissen Feststoff. Die Ausbeute betrug 64% bez. eingesetztem 2-Acetyl-2-azabicyclo[2.2.1]hept-5-en-3-on.
- ¹H-NMR FMSO-d₆) : δ [ppm]: 1,18 (m, 1H);
- 400 MHz: 1,78 (s, 3H); 2,29 (m, 1H); 2,66 (m, 1H); 3,35 (s, 2H); 4,58 (s, 1H); 4,72 (m, 1H); 5,61 (d, 1H); 5,85 (d, 1H); 7,83 (d, 1H).

### Beispiel 8

### Herstellung von (±)-N-Butyryl-1-amino-4-(hydroxymethyl)-2-cyclopenten

73,87 g (±)-2-Butyryl-2-azabicyclo[2.2.1]hept-5-en-3-on wurden unter Stickstoff in Ethanol (400 ml) gelöst und auf -10 °C gekühlt. 15,68 g Natriumborhydrid wurden portionsweise über 45 Minuten zugegeben. Die Reaktion wurde bei 0 °C noch 3 Stunden gerührt und dann wurde der pH auf 1,5 mit konz. Schwefelsäure eingestellt. Dieses Gemisch wurde mit Ethylacetat (200 ml) versetzt und die Feststoffe wurden abfiltriert. Dann wurde es voll eingedampft. Der Rückstand wurde in Wasser aufgenommen, mit Methylenchlorid gewaschen, eingedampft und am Hochvakuum getrocknet. Man erhielt 60,55 g Produkt. Die Ausbeute betrug 80% bez. eingesetztem (±)-2-Butyryl-2-azabicyclo[2.2.1]hept-5-en-3-on.
Smp.: 71 - 72 °C
- ¹H-NMR (CDCl₃) : δ [ppm]: 0,98 (t, J = 8,5 Hz, 3H);
- 400 MHz: 1,40 - 1,50 (1H); 1,58 - 1,68 (2H); 2,10 - 2,18 (2H); 2,42 - 2,55 ( 1H); 2,85 (m, 1H); 3,62 (AB syst., 2H); 4,98 (m, 1H); 5,78-5.82 (2H); 6,38 (m, 1H).

### Beispiel 9

### Herstellung von (±)-N-Phenylacetyl-1-amino-4-(hydroxymethyl)-2-cyclopenten

77 g rohes (±)-2-Phenylacetyl-2-azabicyclo[2.2.1]hept-5-en-3-on wurden unter Stickstoff in Ethanol (450 ml) gelöst und auf -10 °C gekühlt. 13,2 g Natriumborhydrid wurden portionsweise über 1 Stunde zugegeben. Die Reaktion wurde bei Raumtemperatur noch 3,5 Stunden gerührt und dann wurde der pH auf 1,8 mit konz. Schwefelsäure eingestellt. Dieses Gemisch wurde mit einer Kieselgel Filtration (Hexan:Ethylacetat = 2:8) gereinigt. Man erhielt nach Umkristallisation aus Ethylacetat 15,89 g als weissen Feststoff Die Ausbeute betrug 80% bez. eingesetztem (±)-2-Phenylacetyl-2-azabicyclo[2.2.1]hept-5-en-3-on.
- ¹H-NMR (CDCl₃) : δ [ppm]: 1,28 - 1,35 (1H);
- 400 MHz: 1,40 (m, 1H); 2,38 - 2,45 (1H); 2,79 (m, 1H); 3,50 (AB syst., 2H); 3,52 (s, 3H); 4,98 (m, 1H); 5,75 (m, 2H); 5,98 (m, 1H); 7,20 - 7,38 (5H).

### Beispiel 10

### Herstellung von (±)-N-BOC-1-amino-4-(hydroxymethyl)-2-cyclopenten (BOC = tert-Butoxycarbonyl)

15 g rohes (±)-1-Amino-4-(hydroxymethyl)-2-cyclopenten-hydrochlorid (hergestellt gemäss J. Org. Chem. 1981, 46, 3268) wurden unter Stickstoff bei Raumtemperatur in einer Mischung von 150 ml Wasser und 150 ml Dioxan gelöst. Die Lösung wurde mit 1N NaOH auf pH 14 gestellt, dann mit einer Diethylether-Lösung von tert-Butyloxycarbonyl-fluorid (BOC-F, 20% Überschuss) versetzt und bei Zimmertemperatur noch 3 h weitergerührt (BOC-F laut Synthesis 1975, 599 hergestellt). Der pH wurde mit konz. HCl auf 2 gestellt. Nach Destillation von den organischen Lösungsmitteln, wurde zum Rückstand 50 ml Wasser zugegeben und das Gemisch mit 3mal 100 ml Ethylacetat extrahiert. Die vereinigten organischen Phasen wurden vollständig eingedampft. Der Rückstand wurde in einer Mischung von 110 ml Diisopropylether und 80 ml n-Hexan kristallisiert. Man erhielt 11,95 g Titelverbindung. Die Ausbeute betrug 56%.
Smp.: 68 - 70 °C
- ¹H-NMR (DMSO-d₆) : δ [ppm]: 1,18 (m, 1H);
- 400 MHz: 1,38 (s, 9H); 2,26 (m, 1H); 2,65 (m, 1H); 3,33 (t, 2H); 4,45 (m, 1H); 4,55 (t, 1H); 5,62 (m, 1H); 5,79 (m, 1H); 6,73 (d, 1H).

### Beispiel 11

### Herstellung von (±)-N-propionyl-1-amino-4-(hydroxymethyl)-2-cyclopenten

16,6 g (±)-2-Propionyl-2-azabicyclo[2.2.1]hept-5-en-3-on wurden unter Stickstoff in Wasser (140 ml) und 2-Butanol (66 ml) gelöst und auf -5 °C gekühlt. 3 g Natriumborhydrid wurden portionsweise über 2 h zugegeben. Das Gemisch wurde bei 10 °C noch 2,5 h gerührt und dann wurde mit einer Mischung von konz. Salzsäure und Wasser (1/1) auf pH 2,2 eingestellt. Die Lösung wurde auf 40 g eingedampft und auf pH 6,2 mit 2N NaOH eingestellt. Das Gemisch wurde mit 5mal 50 ml Dichlormethan extrahiert. Die vereinigten organischen Phasen wurden vollständig eingedampft und der Rückstand in Toluol (150 ml) umkristallisiert. Man erhielt 11,1 g Titelverbindung. Die Ausbeute betrug 65%.
Smp.: 67 - 68 °C
- ¹H-NMR (DMSO-d₆): δ [ppm]: 0,96 (t, 3H);
- 400 MHz: 1,16 (quint., 1H); 2,04 (quad., 2H); 2,26 (m, 1H); 2,66 (m, 1H); 3,34 (m, 2H); 4,58 (t, 1H); 4,72 (m, 1H); 5,61 (m, 1H); 5,84 (m, 1H); 7,72 (d, 1H).

### Beispiel 12

### Herstellung von (±)-N-Isobutyryl-1-amino-4-(hydroxymethyl)-2-cyclopenten

9 g (±)-2-Isobutyryl-3-azabicyclo[2.2.1]hept-5-en-3-on wurden unter Stickstoff in Wasser (32 ml) und 2-Butanol (84 ml) gelöst und auf 0 °C gekühlt. 1,37 g Natriumborhydrid wurden portionsweise über 3,5 h zugegeben. Das Gemisch wurde bei 20 °C 3 h weitergerührt, dann wurde es mit einer Mischung von konz. Salzsäure und Wasser (1/1) auf pH 2,5 eingestellt und dann mit 2 N NaOH neutralisiert. Die Lösung wurde auf 40 g eingedampft. Der Rückstand wurde mit 3mal 80 ml Dichlormethan extrahiert. Die vereinigten organischen Phasen wurden vollständig eingedampft. Der erhaltene Feststoffwurde in 25 ml Toluol kristallisiert. Man erhielt 6,8 g Titelverbindung. Die Ausbeute betrug 73,6%.
Smp.: 80 - 81 °C
- ¹H-NMR (DMSO-d₆) : δ [ppm]: 0,98 (d, 6H);
- 400 MHz: 1,16 (quint., 1H);
2,30 (m, 2H);
2,68 (m, 1H);
3,32 (t, 2H);
4,58 (t, 1H);
4,70 (m, 1H);
5,61 (m, 1H);
5,82 (m, 1H);
7,68 (d, 1H).

### Beispiel 13

### Herstellung von (1R,4S)-1-Amino-4-(hydroxymethyl)-2-cyclopenten mittels Penicillin G Acylasen

Für die Biotransformation wurde Penicillin G Acylase EC 3.5.1.11 aus E.coli (Boehringer Mannheim) 165 U (Units)/g oder Penicillin G Acylase EC 3.5.1.11 aus Bacillus megaterium eingesetzt. Hierzu wurde 50 mM Natriumphosphatpuffer (pH 5 - 9; 4 ml) mit 1 Gew. % nicht racemisches N-Phenylacetyl-1-amino-4-(hydroxymethyl)-2-cyclopenten und 400 mg der entsprechenden Penicillin G Acylase bei 37 °C inkubiert.
Nach bestimmten Zeitintervallen wurden Proben entnommen und mittels Dünnschichtchromatographie (Kieselgel 60, Butanol:Wasser:Eisessig = 3:1:1; Detektion mit Ninhydrin), Gaschromatographie (Kapillarsäule, HP-5, 5% Phenylmethylsiloxan) oder HPLC analysiert. Das Enzym spaltete mit hoher Aktivität die Phenacetylgruppe ab und setzte dabei bis zu 40% den entsprechenden Aminoalkohol frei. Der freie Aminoalkohol wurde mit einem ee-Wert von 80% erhalten.

### Beispiel 14

### Herstellung von (1R,4S)-1-Amino-4-(hydroxymethyl)-2-cyclopenten mittels Mikroorganismen

14.1 Klärschlamm (20 %) aus der Kläranlage ARA in Visp wurde in dem A + N-Medium (siehe Tabelle 1), enthaltend 0,5 Gew.% N-Acetyl-, N-Propionyl, N-Isobutyryl-, oder N-Butyryl-1-amino-4-(hydroxymethyl)-2-cyclopenten bei 37 °C unter Schütteln inkubiert. Die Bildung von (1R,4S)-1-Amino-4-(hydroxymethyl)-2-cyclopenten wurde mittels Dünnschichtchromatographie verfolgt.
1% dieser Anreicherungen wurden 1-3 mal überimpft und auf Festmedien (Plate Count Agar im Medium von Tabelle 1; 20 g/l) vereinzelt. Auf diese Weise wurden die Mikroorganismen Alcaligenes / Bordetella FB 188 (DSM 1172), Rhodococcus erythropolis CB 101 (DSM 10686), Gordona sp. CB 100 (DSM 10687) und Rhodococcus sp. FB 387 (DSM 11291) isoliert.
14.2 Die auf diese Weise isolierten Mikroorganismen wurden im Medium (Tabelle 1), enthaltend 0,5% N-Acetyl-, N-Propionyl-, N-Isobutyryl oder N-Butyryl-1-amino-4-(hydroxymethyl)-2-cyclopenten gezüchtet. Sie wuchsen 24 bis 36 Stunden bis zu einer optischen Dichte (OD) von 2 bis 3. Die dabei erhaltenen Zellen wurden in der spätexponentiellen Wachstumsphase geerntet und in 10 mM Phosphatpuffer gewaschen.
Die anschliessende Biotransformation wurde in 50 mM Phosphatpuffer (pH 4,5 - 9), enthaltend 1 Gew.% N-Acetyl-, N-Isobutyryl oder N-Butyryl-1-amino-4-(hydroxymethyl)-2-cyclopenten durchgeführt. Nach Dünnschichtchromatographie wurde festgestellt, dass 50 % des Substrates zu (1R,4S)-1-Amino-4-(hydroxymethyl)-2-cyclopenten hydrolysiert waren. HPLC-Analysen ergaben ee-Werte zwischen 80 und 93%.
Wurde als Substrat N-butyryl-1-amino-4-(hydroxymethyl)-2-cyclopenten eingesetzt, betrug die Biotransformationsrate 0,14 (g / 1 / h /OD) für den Stamm DSM 10686, wenn die Anzucht auf A + N-Medium erfolgte und 0,03 (g / 1/ h / OD), wenn die Anzucht auf NYB ("Nutrient Yeast Broth")-Medium, enthaltend N-Butyryl-1-amino-4-(hydroxymethyl)-2-cyclopenten erfolgte.
Wurde die gleiche Umsetzung mit dem Stamm DSM 10687 bei einer Substratkonzentration (N-Butyryl-1-amino-4-(hydroxymethyl)-2-cyclopenten) von 200 mM durchgeführt, betrug die Biotransformationsrate 0,161 (g / 1 / h / OD).

**Tabelle 1**

| **A + N-Medium** | |
|---|---|
| MgCl₂ | 0,4 g/l |
| CaCl₂ | 0,014 g/l |
| FeCl₃ | 0,8 mg/l |
| Na₂SO₄ | 0,1 g/l |
| KH₂PO₄ | 1 g/l |
| Na₂HPO₄ | 2,5 g/l |
| NaCl | 3 g/l |
| Vitaminlösung | 1 ml/l |
| Spurenelementlösung | 1 ml/l |
| pH 7,5 | |

14.3 Rhodococcus erythropolis DSM 10686 wurde in einem 6 l Fermenter in Minimalmedium (vgl. Tabelle 2) mit Ammoniumacetat (3 g/l) als Kohlenstoff- bzw. Stickstoff-Quelle bei 30 °C auf eine Zelldichte von OD 650 > 25 angezogen. Während des Zellwachstums wurde kontinuierlich 50% Essigsäure als zusätzliche C-Quelle zugegeben. Um die enzymatische Aktivität zu induzieren, wurden dann 60 g (+/-) N-Acetyl-1-amino-4-(hydroxymethyl)-2-cyclopenten zugesetzt und für einige Stunden weiter inkubiert. Schliesslich wurden noch einmal 40 g (+/-)-N-Acetyl-1-amino-4-(hydroxymethyl)-2-cyclopenten zugegeben und dann weitere zehn Stunden inkubiert. Der Verlauf der Biotransformation wurde on-line mit HPLC verfolgt. Mit dem Erreichen von 40% analytischer Ausbeute, bezogen auf das eingesetzte racemische Substrat, und einem ee-Wert von 85% wurde die Fermentation durch Säurezugabe abgebrochen.

**Tabelle 2**

| Medienzusammensetzung | |
|---|---|
| **Komponente** | **Konzentration** |
| Hefeextrakt | 0,5 g/l |
| Pepton M66 | 0,5 g/l |
| KH₂PO₄ | 4,0 g/l |
| Na₂HPO₄.2H₂O | 0,5 g/l |
| K₂SO₄ | 2,0 g/l |
| NH₄-acetat | 3,0 g/l |
| CaCl₂ | 0,2 g/l |
| MgCl₂·6H₂O | 1,0 g/l |
| Spurenelemente-Lösung (siehe unten) | 1,5 ml/l |
| PPG (Polypropylenglycol) | 0,1 g/l |

| **Spurenelemente-Lösung** | |
|---|---|
| KOH | 15,1 g/l |
| EDTA·Na₂·2H₂O | 100,0 g/l |
| ZnSO₄·7H₂O | 9,0 g/l |
| MnCl₂·4H₂O | 4,0 g/l |
| H₃BO₃ | 2,7 g/l |
| CoCl₂·6H₂O | 1,8 g/l |
| CuCl₂·2H₂O | 1,5 g/l |
| NiCl₂·6H₂O | 0,18 g/l |
| Na₂MoO₄·2H₂O | 0,27 g/l |

14.4 Analog zu Beispiel 14.3 wurden die Mikroorganismen Arthrobacter sp. HSZ 5 (DSM 10328), Rhodococcus sp FB387 (DSM 11291), Alcaligenes xylosoxydans ssp. denitrificans HSZ 17 (DSM 10329), Agrobacterium / Rhizobium HSZ 30, Bacillus simplex K2 und Pseudomonas putida K32 auf Natriumacetat im Medium (Tabelle 1) mit und ohne N-Acetyl-, N-Propionyl, N-Isobutyryl- oder N-Butyryl-1-amino-4-(hydroxymethyl)-2-cyclopenten, im folgenden als Aminoalkohole abgekürzt, angezogen.
Folgende Ergebnisse wurden mit exponentiellen Zellen, die ohne Aminoalkohole angezogen wurden, erzielt (HPLC-analysiert):

| **Stamm** | **Rate [mmol/OD.h]** | **ee-Wert/Umsatz [%]** |
|---|---|---|
| HSZ 5 (DSM 10328) | 0,05 | 88,7/16 |
| HSZ 17 (DSM 10329) | 0,005 | 95/23 |
| K32 | 0,05 | 54/1 |
| CB101 (DSM 10686) | 0,1 | 84/39 |

Die Stämme K2 und HSZ 30 wurden angezogen, geerntet und einer 60-stündigen Biotransformation unterzogen.

| **Stamm** | **Rate [mmol/OD.h]** | **ee-Wert/Umsatz [%]** |
|---|---|---|
| K2 | - | 92/10 |
| HSZ 30 | - | 93/3,5 |

Von allen Ansätzen wurden exponentielle und stationäre Zellen geerntet und als ruhende Zellen zur Biotransformation eingesetzt. Es konnte aufgrund der DC-Analyse kein Unterschied von mit Aminoalkohol induzierten oder nicht induzierten Zellen in der Anfangsrate beobachtet werden.

### Beispiel 15

### Reinigung der N-Acetylaminoalkoholhydrolase aus Rhodococcus erythropolis CB101 (DSM 10686)

Das Enzym wurde wie nachstehend beschrieben gereinigt bis nur noch eine Proteinbande im SDS-PAGE (Pharmacia Phast Gel, 10-15 % Gradient) bei einem Molekulargewicht von 50 kD beobachtet wurde.

Zellen von Rhodococcus erythropolis CB101 (DSM 10686) wurden in 50 mM Tris-Puffer (pH 6,2) gewaschen und auf eine optische Dichte von 190 bei OD₆₅₀ₙₘ konzentriert. Nach Zugabe von Phenylmethansulfonylfluorid (PMSF) auf eine Endkonzentration von 1 mM und DNAse wurden die Zellen mit der French-Press behandelt, um ein Rohextrakt zu erhalten. Nach Zentrifugieren wurden 200 ml zellfreies Extrakt mit einer Proteinkonzentration von 4,8 mg ml⁻¹ erhalten.
960 mg des zellfreien Extraktes wurden auf eine HiLoad 26/10 Q-Sepharose-Ionenaustausch-Chromatographie-Säule (Pharmacia) aufgetragen, die mit einem 50 mM Tris-Puffer (pH 8,0), enthaltend 1 mM Dithiothreitol (DTT), equilibriert worden war.
Nach Waschen der Säule mit dem gleichen Puffer wurden die Proteine mit einem linearen Puffer-Gradienten (1500 ml; Gradient: 50 mM Tris-Puffer (pH 8,0), enthaltend 1 mM DTT - 50 mM Tris-Puffer (pH 7,0), enthaltend 1 mM DTT und 1 M NaCl) eluiert. Das Enzym eluierte von der Säule zwischen 370 und 430 mM NaCl und bei einem pH von 7,6. Die aktiven Fraktionen wurden gesammelt und auf 9 ml konzentriert. Der Proteingehalt betrug 41 mg.
Zur weiteren Reinigung wurde die Proteinlösung auf eine Gelfiltrationschromatographie-Säule HiLoad 26/60 Superdex 75 (Pharmacia) aufgetragen, die mit einem 50 mM Tris-Puffer, enthaltend 50 mM NaCl und 1 mM DTT, equlibriert worden war. Die aktiven Fraktionen wurden vereinigt und hatten einen Gesamtproteingehalt von 10,9 mg.
Diese Proteinlösung wurde auf eine Mono Q HR5/5 Säule (Pharmacia) aufgetragen, die mit 50 mM Tris-Puffer (pH 8,5), enthaltend 1 mM DTT, equilibriert worden war. Die Proteine wurden mit einem linearen Gradienten (40 ml) von 50 mM Tris-Puffer (pH 8,5), enthaltend 1 mM DTT - 50 mM Tris-Puffer (pH 8,5), enthaltend 1 mM DTT und 1 M NaCl eluiert. Das Enzym eluierte zwischen 390 mM NaCl und 440 mM NaCl. Die aktiven Fraktionen enthielten 1,4 mg Protein.
Im letzten Reinigungsschritt wurde die gleiche Säule, equilibriert mit dem gleichen Puffer, verwendet. Als Elutionsgradient diente dergleiche Puffer mit 0 - 500 mM NaCl und pH 7,0 - 8,5. Auf diese Weise konnten 430 µg reines Enzym isoliert werden.

Die N-terminale Sequenz des Enzyms wurde direkt vom "Protein-Blot" bestimmt. Es wurde eine Sequenz von den 20 folgenden Aminosäuren erhalten (vgl. WO-A-97/45529):

### Beispiel 16

### Enzymcharakterisierung

Die Enzymcharakterisierung wurde sowohl mit gereinigtem Enzym als auch mit zellfreiem Extrakt, das mittels einer Sephadex G-25 Säule (PD-10, Pharmacia) entsalzt war, durchgeführt.
Die Proteinkonzentration im zellfreien Extrakt betrug 7,3 mg ml⁻¹ und die Proteinkonzentration des gereinigten Enzyms betrug 135 µg ml⁻¹. PMSF wurde zum zellfreien Extrakt nicht zugegeben.
16.1 Kₘ-Bestimmung
   Die Kₘ-Bestimmung wurde im zellfreien Extrakt durchgeführt. Der Kₘ-Wert der Reaktion betrag bei pH 7,0 und bei einer Temperatur von 30 °C 22,5 mM für das Substrat N-Acetyl-1-amino-4-(hydroxymethyl)-2-cyclopenten.
16.2 pH-Optimum
   Das pH-Optimum für die Hydrolyse von N-Acetyl-1-amino-4-(hydroxymethyl)-2-cyclopenten (25 mM) wurde mit dem gereinigten Enzym und in zellfreiem Extrakt in einem pH-Bereich von pH 6,2 - 9,0 in folgenden Pufferlösungen bestimmt.
   Tris-Puffer 100 mM pH 9,0; 8,5; 8,0; 7,5; 7,0
   Citrat-Phosphat-Puffer 100 mM pH 7,0; 6,55; 6,2
   Die Aktivität wurde 24 h lang gemessen.
   Das pH-Optimum für die Reaktion war zwischen pH 7,0 und pH 7,5 für die Produktion des (1R,4S)- und des (1S,4R)-Enantiomeren.
   Im zellfreien Extrakt lag das pH-Optimum für die Aktivität bei pH 7,0. Die Selektivität war jedoch besser zwischen pH 6,0 und pH 7,0.

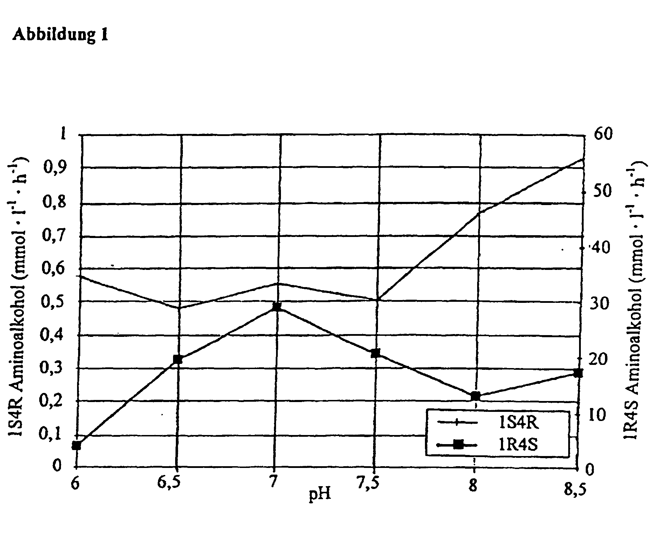

   Abbildung 1 zeigt die Aktivität der N-Acetylaminoalkoholhydrolase (zellfreies Extrakt) aus Rhodococcus erythropolis CB 101 (DSM 10686) in Abhängigkeit vom pH-Wert.
16.3 Das Temperaturoptimum für die in Beispiel 16.2 angegebene Reaktion lag zwischen 25 und 30 °C.

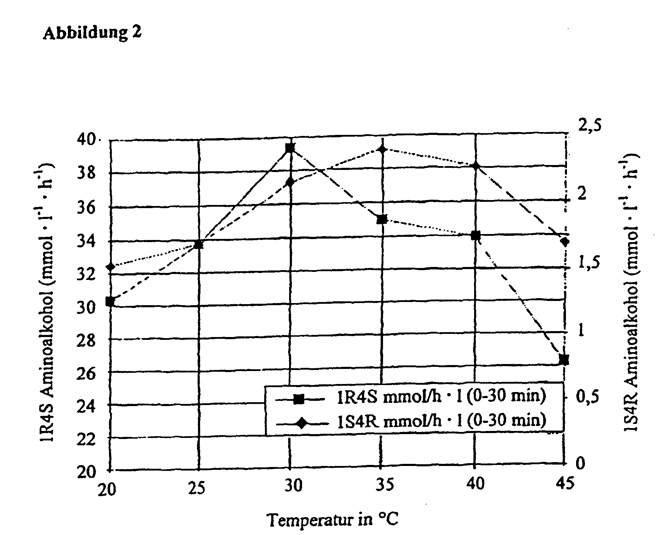

   Abbildung 2 zeigt die Aktivität der N-Acetylaminoalkoholhydrolase (zellfreies Extrakt) aus Rhodococcus erythropolis CB 101 (DSM 10686) in Abhängigkeit von der Temperatur.
16.4 Das Molekulargewicht wurde zu 50 kD gemäss SDS-PAGE bestimmt.
16.5 Als Substrate wurden hydrolysiert: N-Acetyl-1-amino-4-hydroxymethyl-2-cyclopenten, N-Butyryl-1-amino-4-(hydroxymethyl)-2-cyclopenten, N-propionyl-1-amino-4-(hydroxymethyl)-2-cyclopenten, N-Isobutyryl-1-amino-4-(hydroxymethyl)-2-cyclopenten, N-Isobutyl-1-amino-4-(hydroxymethyl)-2-cyclopenten.

### Beispiel 17

### Herstellung von (1R,4S)-1-Amino-4-(hydroxymethyl)-2-cyclopenten-hydrochlorid

374,1 g einer Lösung von (1R,4S)-1-Amino-4-(hydroxymethyl)-2-cyclopenten (ähnliche Herstellung wie beim Beispiel 14) wurde auf 123,7 g eingedampft. Die Lösung enthielt 60,2 mMol der obigen Verbindung (HPLC) und wurde mit 30%iger NaOH von pH 2 auf pH 11,7 gestellt, dann mit 3mal 70 ml Isobutanol extrahiert. Die vereinigten Isobutanol-Extrakte wurden mit gasförmigen HCl auf pH 1 gestellt, auf 65 g aufkonzentriert und filtriert (Entfernung von festen Verunreinigungen). Zum Filtrat wurden, bei 20 °C und unter guter Rührung, 60 ml Aceton zugetropft. Die trübe Mischung wurde mit Kristallen der Titelverbindung angeimpft und 1 h bei 5 °C gerührt. Nach Filtration und Trocknung erhielt man 5,2 g Produkt. Die Ausbeute betrug 58%.
Smp.: 125 -127 °C
ee-Wert 98% (geeichte chirale HPLC-Säule)
- ¹H-NMR (DMSO-d₆) : δ [ppm]: 1,44 (m, 1H);
- 400 MHz: 2,35 (m, 1H);
2,83 (m, 1H);
3,42 (m, 2H);
4,10 (s, 1H);
4,80 (s, 1H);
5.80 (d, 1H);
6,06 (d, 1H);
8,13 (s, 3H).

### Beispiel 18

### Herstellung von (1R,4S)-N-BOC-1-amino-4-(hydroxymethyl)-2-cyclopenten

75 g einer Lösung von (1R,4S)-1-Amino-4-(hydroxymethyl)-2-cyclopenten (ähnliche Herstellung wie beim Beispiel 14; 44,6 mMol der Verbindungen) wurde mit 30%igen NaOH auf pH 8 gestellt und mit 6 g NaHCO₃ versetzt. Das Gemisch wurde auf 52 °C erwärmt. Unter guter Rührung wurde dazu 60 ml Diisopropylether zugegeben und dann während 2 h eine Lösung von 11,12 g BOC-Anhydrid in 18,2 ml Diisopropylether zudosiert. Die Mischung wurde über Celite filtriert und die Phasen getrennt. Die Wasserphase wurde mit 65 ml Diisopropylether extrahiert. Die vereinigten organischen Phasen wurden mit 45 ml Wasser gewaschen, dann auf 37,5 g eingedampft und auf 50 °C erwärmt. Zu der Lösung wurde 31 ml n-Hexan zugetropft. Nach langsamer Kühlung auf 0 °C (2 h), wurde die Titelverbindung filtriert, mit 12 ml n-Hexan / Diisopropylether 1/1 gewaschen und getrocknet. Man erhielt 6,75 g Produkt. Die Ausbeute betrug 71%.
Smp.: 70 - 71 °C
ee-Wert 98% (geeichte chirale HPLC-Säule)
- ¹H-NMR (DMSO-d₆) : δ [ppm]: 1,18 (m, 1H);
- 400 MHz: 1,27 (s, 9H);
2,28 (m, 1H);
2,63 (m, 1H);
3,33 (q, 2H);
4,43 (m, 1H);
4,56 (t, 1H);
5,62 (m, 1H);
5,78 (m, 1H);
6,72 (d, 1H).

### Beispiel 19

### Herstellung von (1R,4S)-1-Amino-4-(hydroxymethyl)-2-cyclopenten-hydrochlorid

87,8 g (1R,4S)-N-BOC-1-Amino-4-(hydroxymethyl)-2-cyclopenten wurden in 270 ml 2N HCl und 1340 ml Methanol gelöst. Das Gemisch wurde unter Rückfluss 4.5 h gekocht. Nach Destillation des Methanols wurde der Rückstand in 800 ml Wasser aufgelöst. Die wässrige Lösung wurde 2mal mit 340 ml Ethylacetat extrahiert. Die Wasserphase wurde vollständig eingedampft (50 °C / 60 mbar). Der Feststoffwurde unter Vakuum bei 50 °C getrocknet, mit 150 ml Diethylether aufgeschlämmt, abfiltriert und 2mal mit 50 ml Diethylether gewaschen. Nach Trocknung wurde die Titelverbindung mit 95% Ausbeute erhalten (58,4 g).

Die physikalischen und spektroskopischen Daten des Produktes waren gleich wie bei Beispiel 17.

### Beispiel 20

### Herstellung von (1R,4S)-N-Acetyl-1-amino-4-(hydroxymethyl)-2-cyclopenten

25 g (1R,4S)-1-Amino-4-(hydroxymethyl)-2-cyclopenten-hydrochlorid wurden in 182 ml Essigsäureanhydrid gelöst und bei 0 °C mit einer Lösung von 18,25 g Triethylamin in 60 ml Essigsäureanhydrid versetzt. Die Mischung wurde bei 80 °C 3 h gerührt, dann auf Raumtemperatur abgekühlt. Das Triethylaminhydrochlorid wurde abfiltriert und mit 120 ml n-Hexan gewaschen. Das Filtrat wurde eingedampft. Der Rückstand wurde mit 300 ml Toluol versetzt und bei Raumtemperatur in Gegenwart von 5,2 g Aktivkohle und 13 g Celite 20 Min. gerührt. Nach Filtration und Waschen des Filterkuchens (3mal 40 ml Toluol), wurde das Lösungsmittel vollständig eingeengt. Der Rückstand wurde mit 180 ml Methanol und 15,5 g K₂CO₃ versetzt und bei Raumtemperatur 10 h gerührt. Die Suspension wurde abfiltriert und das Filtrat eingedampft. Der Rückstand wurde in 750 ml Isopropylacetat suspendiert und in Gegenwart von 0,5 g Aktivkohle auf Rückfluss 1,5 h abgekocht. Nach der Aktivkohle-Filtration (70 - 80 °C) wurde das Filtrat über Nacht bei 0 °C gekühlt. Die Titelverbindung wurde filtriert, mit 80 ml kalten Isopropylacetat gewaschen und unter Vakuum getrocknet. Man erhielt 17,2 g Produkt. Die Ausbeute betrug 66%.
Smp.: 77 - 80 °C
ee-Wert 98% (geeichte chirale HPLC-Säule)
- ¹H-NMR (DMSO-d₆): δ [ppm]: 1,15 (m, 1H);
- 400 MHz: 1,78 (s, 3H);
2,25 (m, 1H);
2,66 (m, 1H);
3,35 (m, 2H);
4,58 (t, 1H);
4,70 (m, 1H);
5,62 (m, 1H);
5,85 (m, 1H);
7,80 (d, 1H).

### Beispiel 21

### Herstellung von (1S,4R)-N-Acetyl-1-amino-4-(hydroxymethyl)-2-cyclopenten

Ausgehend von 25 g (1S,4R)-1-Amino-4-(hydroxymethyl)-2-cyclopenten-hydrochlorid konnte das Titelenantiomer mit dem Verfahren gemäss Beispiel 18 hergestellt werden (Ausbeute 68%). Die spektroskopischen und physikalischen Daten des Produktes waren gleich wie bei Beispiel 20.

### Beispiel 22

### Herstellung von (1R,4S)-N-Butyryl-1-amino-4-(hydroxymethyl)-2-cyclopenten

34,7 g(1R,4S)-1-Amino-4-(hydroxymethyl)-2-cyclopenten-hydrochlorid und 2 g 4-N,N-dimethylaminopyridin wurden in 600 ml Methylenchlorid gelöst. Die Lösung wurde auf 0 °C gekühlt. Dann wurden 52 g Triethylamin zugetropft (5 Min.). Das Gemisch wurde noch 30 Min. weitergerührt. Zu der Mischung wurde bei 0 °C eine Lösung von 35,2 g Butyrylchlorid in 60 ml Methylenchlorid während 1 h zudosiert. Das Gemisch wurde noch zwischen 0 bis 20 °C 1,5 h weitergerührt, dann mit 600 ml Wasser versetzt. Nach Phasentrennung wurde die Wasserphase mit 600 ml Methylenchlorid extrahiert. Die vereinigten organischen Phasen wurden 3mal mit je 500 ml 10%iger NaOH gewaschen, dann vollständig eingedampft. Der getrocknete Feststoff wurde in 120 ml Methanol gelöst. Die Lösung wurde mit 5 g K₂CO₃ versetzt und bei Raumtemperatur 2 h weitergerührt. Die anorganischen Salze wurden abfiltriert und mit 20 ml Methanol gespült. Das Filtrat wurde mit 2N HCl neutralisiert. Die Suspension wurde abfiltriert und der Filterkuchen mit 20 ml Methanol gewaschen. Das Filtrat wurde vollständig eingedampft. Der feste Rückstand wurde getrocknet und in 150 ml Toluol kristallisiert. Man erhielt 28,5 g Titelverbindung. Die Ausbeute betrug 67%.
Smp.: 71 - 72 °C
ee-Wert 98% (geeichte chirale HPLC-Säule)
- ¹H-NMR (DMSO-d₆) : δ [ppm]: 0,85 (t, 3H);
- 400 MHz: 1,15 (m, 1H);
1,50 (q, 2H);
2,03 (d, 2H);
2,28 (m, 1H);
2,67 (m, 1H);
3,35 (d, 2H);
4,62 (s, 1H);
4,76 (m, 1H);
5,63 (m, 1H);
5,85 (m, 1H);
7,77 (d, 1H).

### Beispiel 23

### Herstellung von (1S,4R)-N-Butyryl-1-amino-4-(hydroxymethyl)-2-cyclopenten

Ausgehend von 34,7 g (1 S,4R)-1-Amino-4-(hydroxymethyl)-2-cyclopenten-hydrochlorid konnte das Titelenantiomer mit dem Verfahren von Beispiel 20 hergestellt werden (Ausbeute 63%). die spektroskopischen und physikalischen Daten des Produktes waren gleich wie bei Beispiel 22.

### Beispiel 24

### Herstellung von (1R,4S)-1-[(2-Amino-6-chlor-5-formamidopyrimidin-4-yl)amino]-4-(hydroxymethyl)-2-cyclopenten

2,07 g N-(2-Amino-4,6-dichlorpyrimidin-5-yl)formamid wurden in 40 ml Isobutanol auf 80 °C erwärmt (weisse Suspension). Zu der Mischung wurde eine Lösung von 1,97 g (1R,4S)-1-amino-4-(hydroxymethyl)-2-cyclopenten-hydrochlorid, 3,8 g Triethylamin und 15 ml Isobutanol hinzugegeben. Das Gemisch wurde bei 80 °C 13 h weitergerührt. Die klare Lösung wurde bei 20 °C mit 10 ml 1N NaOH versetzt und bis zur Trockene eingedampft. Der Rückstand wurde flash-chromatographiert (Kieselgel 60-Säule, Länge 8 cm, Durchmesser 6,5 cm, Fliessmittel Ethylacetat / Methanol 95/5). Nach dem Eindampfen des Fliessmittels und der Trocknung des Rückstandes erhielt man 2,1 g der Titelverbindung. Die Ausbeute betrug 74%.
Smp.: 174-176 °C
ee-Wert 98% (geeichte chirale HPLC-Säule)
- ¹H-NMR (DMSO-d₆) : δ [ppm]: 1,37 (m, 1H);
- 400 MHz: 2,35 (m, 1H);
2,73 (m, 1H);
3,38 (t, 2H);
4,68 (m, 1H);
5,08 (m, 1H);
5,70 (d, 1H);
5,85 (d, 1H);
6,40; 6,55 und 6,65 (s, d, und d, zusammen 3H);
7,78 und 8,10 (d und s, zusammen 1H);
8,55 und 8,95 (d und s, zusammen 1H).

### Beispiel 25

### Herstellung von (1R,4S)-1-[(2-Amino-6-chlor-5-formamidopyrimidin-4-yl)amino]-4-(hydroxymethyl)-2-cyclopenten

145,2 ml einer Lösung von (1R,4S)-1-Amino-4-(hydroxymethyl)-2-cyclopenten (ähnliche Herstellung wie in Beispiel 14) wurde auf 25,5 ml konzentriert und über Celite abfiltriert. Der Filterkuchen wurde mit 7,5 ml Wasser gewaschen. Das Filtrat enthielt 17,7 mMol der obigen Verbindung (HPLC) und wurde mit konz. HCl von pH 6,6 auf pH 1 gestellt, dann 3mal mit 20 ml Isobutanol extrahiert. Die organische Phase wurde weggeworfen. Die wässrige Phase wurde mit 30%iger NaOH auf pH 12 gestellt und 3mal mit 10 ml Isobutanol extrahiert. Die vereinigten organischen Phasen wurden auf 15 ml eingedampft und mit 2,53 g Triethylamin versetzt. Die Mischung wurde, wie bei Beispiel 24 mit einer Lösung von 2,07 g N-(2-amino-4,6-dichlorpyrimidin-5-yl)formamid in 40 ml Ethanol versetzt. Das Gemisch wurde bei 80 °C 16 h gerührt. Nach der Aufarbeitung wie bei Beispiel 22 erhielt man 2,4 g Titelverbindung. Die Ausbeute betrug 85%.
Die physikalischen und spektroskopischen Daten des Produktes waren gleich wie bei Beispiel 24.

### Beispiel 26

### Herstellung von (±)-N-BOC-1-amino-2-cyclopenten-4-carbonsäure

16,4 g rohes (±)-1-Amino-2-cyclopenten-4-carbonsäure-hydrochlorid (hergestellt gemäss J. Org. Chem. 1981, 46, 3268) wurden unter Stickstoff bei Zimmertemperatur in einer Mischung von 80 ml Wasser und 80 ml Dioxan gelöst. Die Mischung wurde dann mit 1N NaOH auf pH 14 gestellt und mit einer Diethylether-Lösung von tert-Butyloxycarbonyl-fluorid (BOC-F, 20% Überschuss) versetzt (BOC-F laut Synthesis 1975, 599 hergestellt). Das Gemisch wurde bei Zimmertemperatur noch 5 h weitergerührt. Der pH wurde mit konz. HCl auf 2 gestellt. Nach der Destillation von den organischen Lösungsmitteln wurde der Rückstand mit 50 ml Wasser addiert und 3mal mit 100 ml Ethylacetat extrahiert. Die organische Phase wurde auf 50 ml eingedampft und mit 25 ml Toluol verdünnt. Nach Kühlung (0 - 10 °C) wurde die Titelverbindung filtriert und getrocknet. Man erhielt 14,3 g Produkt. Die Ausbeute betrug 63%.
Smp.: 126-127°C
- ¹H-NMR (DMSO-d₆) : δ [ppm]: 1,14 (s, 9H);
- 400 MHz: 1,70 (m, 1H);
2,40 (m, 1H);
3,40 (m, 1H);
4,47 (m, 1H);
5,70 (t, 1H);
4,87 (t, 1H);
6,88 (d, 1H);
12,30 (d, 1H).

### Beispiel 27

### Herstellung von (±)-N-BOC-1-amino-4-(hydroxymethyl)-2-cyclopenten aus (±)-N-BOC-1-amino-2-cyclopenten-4-carbonsäure

In einem 500 ml Rührwerk wurden 250 ml Tetrahydrofuran und 10,02 g (0,164 mol) LiAlH₄ vorgelegt und eine Lösung von 30,0 g (0,132 mol) (±)-N-BOC-1-amino-2-cyclopenten-4-carbonsäure in 75 ml Tetrahydrofuran bei -10 °C innerhalb von 1 h zudosiert. Anschliessend wurden 10 g Wasser, 10 g 15%ige NaOH-Lösung und 20 g Wasser zugegeben und abfiltriert. Der Rückstand wurde noch 2 mal mit je 100 ml tert. Butylmethylether gewaschen und die vereinigten organischen Phasen wurden zur Trockne eingedampft. Nach dem Zusatz von 80 ml Hexan und Animpfen mit der Verbindung gemäss Beispiel 10 fiel die Titelverbindung als kristalliner Feststoff aus. Ausbeute 15,84 g (56%). Die physikalischen und spektroskopischen Daten waren gleich wie in Beispiel 10.

### Beispiel 28

### Herstellung von (1R,4S)-N-BOC-1-amino-4-(hydroxymethyl)-2-cyclopenten aus (1R,4S)-N-BOC-1-amino-2-cyclopenten-4-carbonsäure

In einem 500 ml Rührwerk wurden 80 ml Tetrahydrofuran und 11,38 g (50,07 mmol) (1R,4S)-N-BOC-1-amino-2-cyclopenten-4-carbonsäure (hergestellt gemäss Tetrahedron: Asymmetry 1993, 4, 1117) vorgelegt und 5 ml Boran-dimethylsulfid-Komplex innerhalb von 1 h bei -15 °C zudosiert und anschliessend noch 3 h bei dieser Temperatur nachgerührt. Eine Lösung von 4 g NaOH in 60 ml Wasser wurde zugegeben und auf Raumtemperatur aufgewärmt. Extraktion mit Toluol, Filtration über Kieselgel und anschliessende Kristallisation in Ethylacetat / n-Hexan 1:1 lieferte 5,4 g der Titelverbindung, entsprechend einer Ausbeute von 57%, als weissen kristallinen Feststoff. Die physikalischen und spektroskopischen Daten waren gleich wie in Beispiel 10. Der ee-Wert betrug 99% (gleiche chirale HPLC-Säule).

## Patentansprüche

1. Verfahren zur Herstellung von (1S,4R)- und/oder (1R,4S)-4-(2-Amino-6-chlor-9-Hpurin-9-yl)-2-cyclopenten-1-methanol der Formeln I oder II 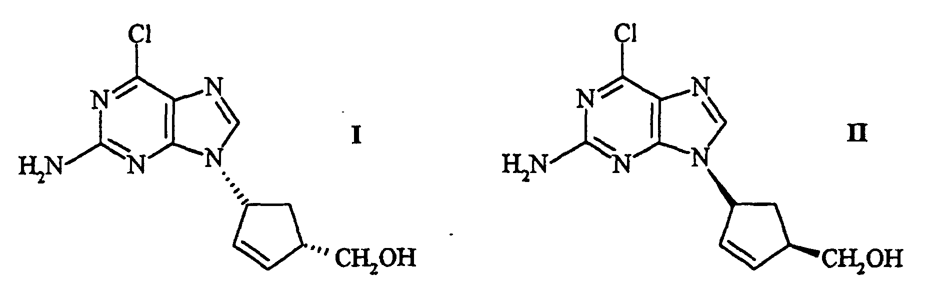 oder deren Salzen, **dadurch gekennzeichnet, dass** man in einer ersten Stufe ein (±)-2-Azabicyclo[2.2.1]hept-5-en-3-on der Formel 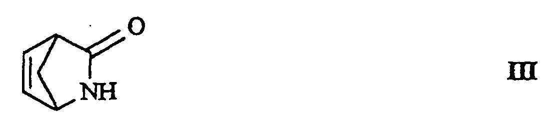 zu einem (±)-2-Azabicyclo[2.2.1]hept-5-en-3-on-derivat der allgemeinen Formel 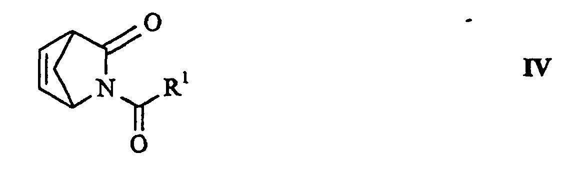 worin R¹ C₁₋₄-Alkyl, C₁₋₄-Alkoxy, Aryl oder Aryloxy bedeutet, acyliert, dieses in einer zweiten Stufe zu einem 1 Amino-4-(hydroxymethyl)-2-cyclopentenderivat der allgemeinen Formel 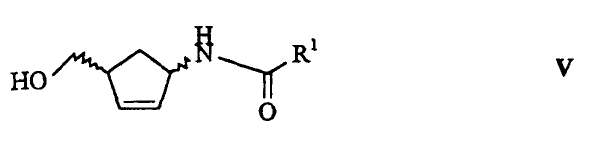 worin R¹ die genannte Bedeutung hat, reduziert, dieses in einer dritten Stufe mittels eines Mikroorganismus, der befähigt ist, Cyclopentanderivate der allgemeinen Formel V als als einzige Stickstoff-, als einzige Kohlenstoff- oder als einzige Kohlenstoff- und Stickstoffquelle zu verwerten, Enzymextrakten daraus, einem Enzym mit N-Acetylaminoalkoholhydrolase-Aktivität oder einer Penicillin G Acylase zum (1R,4S)-oder (1S,4R)-1-Amino-4-(hydroxymethyl)-2-cyclopenten gemäss den Formeln 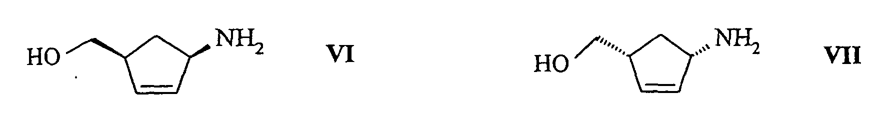 umsetzt, dieses, gegebenenfalls nach Auftrennung der Enantiomeren, in einer vierten Stufe mit N-(2-amino-4,6-dichlorpyrimidin-5-yl)formamid der Formel 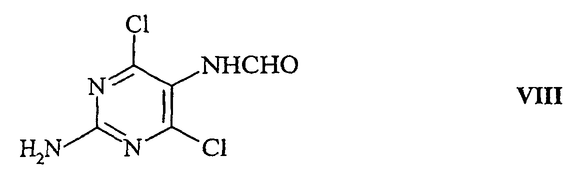 in das (1S,4R)- oder (1R,4S)-4-[(2-amino-6-chlor-5-formamido-4-pyrimidinyl)-amino]-2-cyclopenten-1-methanol der Formeln 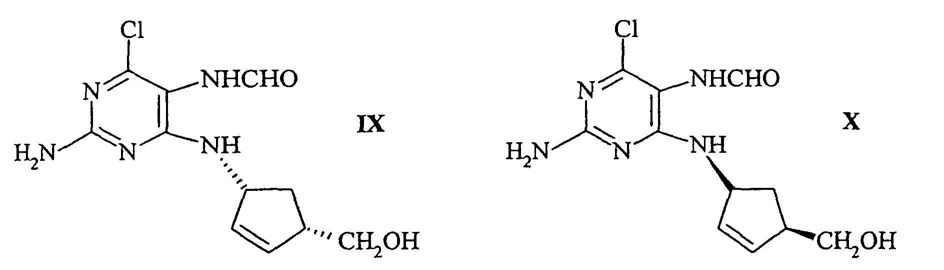 überführt und letztere in einer fünften Stufe zum Endprodukt gemäss den Formeln I oder II cyclisiert.

2. Verfahren nach Anspruch 1, **dadurch gekennzeichnet, dass** man die Acylierung in der ersten Stufe mit einem Carbonsäurehalogenid der allgemeinen Formel 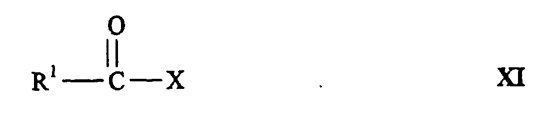 worin R¹ die genannte Bedeutung hat und X ein Halogenatom bedeutet, oder mit einem Carbonsäureanhydrid der allgemeinen Formel 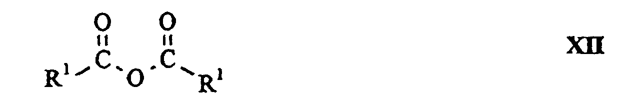 worin R¹ die genannte Bedeutung hat,durchführt.

3. Verfahren nach einem der Ansprüche 1 oder 2, **dadurch gekennzeichnet, dass** man die Acylierung in der ersten Stufe in einem aprotischen Lösungsmittel durchführt.

4. Verfahren nach einem der Ansprüche 1 bis 3, **dadurch gekennzeichnet, dass** man die Reduktion in der zweiten Stufe mit einem Alkali- oder Erdalkalimetallborhydrid, einem Alkali- oder Erdalkalimetallaluminiumhydrid oder mit einem Vitrid durchführt.

5. Verfahren nach einem der Ansprüche 1 bis 4, **dadurch gekennzeichnet, dass** man die Reduktion in der zweiten Stufe in einem protischen Lösungsmittel durchführt.

6. Verfahren nach einem der Ansprüche 1 bis 5, **dadurch gekennzeichnet, dass** man die Umsetzung in der dritten Stufe mittels Mikroorganismen der Gattung Rhodococcus, Gordona, Arthrobacter, Alcaligenes, Agrobacterium / Rhizobium, Bacillus, Pseudomonas oder Alcaligenes / Bordetella durchführt.

7. Verfahren nach einem der Ansprüche 1 bis 5, **dadurch gekennzeichnet, dass** man die Umsetzung in der dritten Stufe mittels einer Penicillin G Acylase aus Mikroorganismen der Spezies Bacillus megaterium oder Escherichia coli durchführt.

8. Verfahren nach einem der Ansprüche 1 bis 7, **dadurch gekennzeichnet, dass** man die Umsetzung in der dritten Stufe bei einer Temperatur von 20 bis 40 °C und bei einem pH-Wert von 5 bis 9 durchführt.

9. Verfahren nach einem der Ansprüche 1 bis 8, **dadurch gekennzeichnet, dass** man die Umsetzung in der vierten Stufe in Gegenwart einer Base durchführt.

10. Verfahren nach einem der Ansprüche 1 bis 9, **dadurch gekennzeichnet, dass** man die Umsetzung in der vierten Stufe in einem protischen Lösungsmittel durchführt.

11. Verfahren zur Herstellung von (1R,4S)- und/oder (1S,4R)-1-Amino-4-(hydroxymethyl)-2-cyclopentenderivaten der allgemeinen Formeln XVI oder XVII 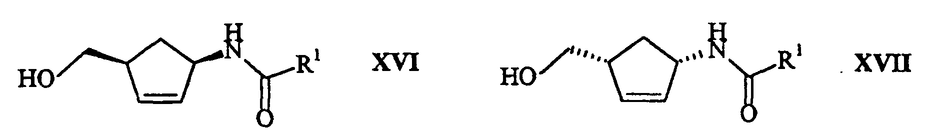 worin R¹ C₁₋₄-Alkyl, C₁₋₄-Alkoxy, Aryl oder Aryloxy bedeutet, **dadurch gekennzeichnet, dass** man ein Cyclopentenderivat der allgemeinen Formel 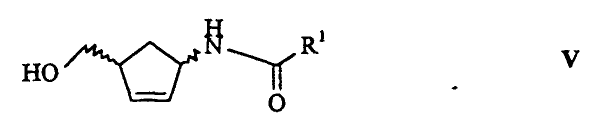 worin R¹ die genannte Bedeutung hat, mittels eines Mikroorganismus, der befähigt ist, Cyclopentanderivate der allgemeinen Formel V als einzige Stickstoff-, als einzige Kohlenstoff- oder als einzige Kohlenstoff- und Stickstoffquelle zu verwerten, Enzymextrakten daraus, einem Enzym mit N-Acetylaminoalkoholhydrolase-Aktivität oder einer Penicillin G Acylase zum (1R,4S)- und (1S,4R)-1-Amino-4-(hydroxymethyl)-2-cyclopenten gemäss den Formeln 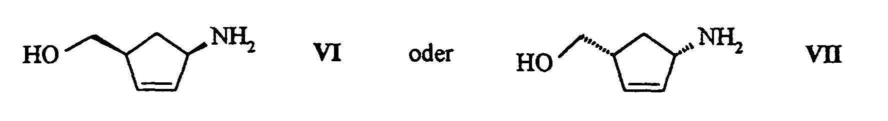 umsetzt und dieses, gegebenenfalls nach Auftrennung der Enantiomeren, zu den Verbindungen der Formeln XVI oder XVII acyliert.

12. (1R,4S)-N-Butyryl-1-amino-4-(hydroxymethyl)-2-cyclopenten der Formel 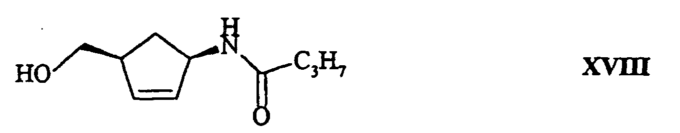 im Enantiomerenüberschuß.

13. Enantiomer nach Anspruch 12 mit einem Enantiomerenüberschuß von mindestens 80 %.

14. Enantiomer nach Anspruch 12 mit einem Enantiomerenüberschuß von mindestens 98 %.

## Claims

1. Process for the production of (1S,4R)- and/or (1R/4S)-4-(2-amino-6-chloro-9-H-purin-9-yl)-2-cyclopentene-1-methanol of the formulae I or II 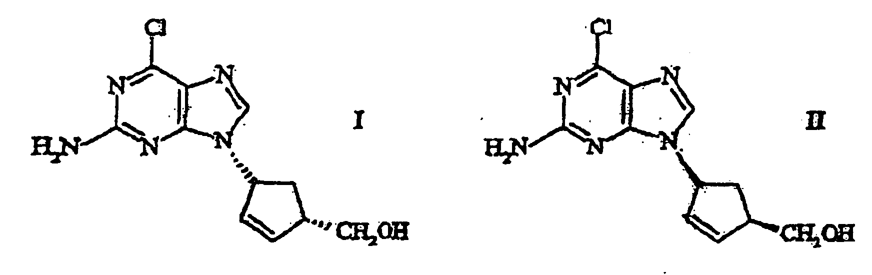 or salts thereof, **characterised in that** in a first stage a (±)-2-azabicyclo[2.2.1]hept-5-en-3-one of the formula 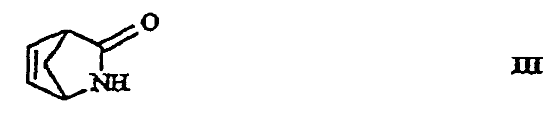 is acylated to a (±)-2-azabicyclo[2.2.1]hept-5-en-3-one derivative of the general formula 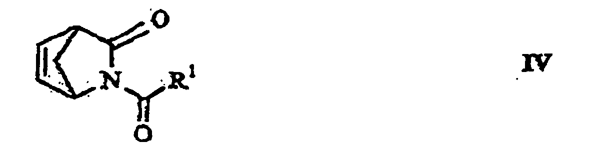 in which R¹ means C₁₋₄-alkyl, C₁₋₄-alkoxy, aryl or aryloxy, this is reduced in a second stage to a 1-amino-4-(hydroxymethyl)-2-cyclopentene derivative of the general formula 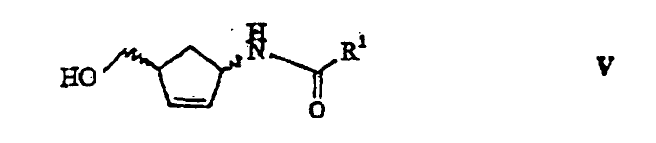 in which R¹ has the given meaning, this is converted in a third stage by means of a microorganism that is capable of using cyclopentane derivatives of the general formula V as the sole nitrogen-, the sole carbon- or the sole carbon- and nitrogen source, enzyme extracts thereof, of an enzyme having N-acetylamino alcohol hydrolase activity or of a penicillin G acylase, to (1R,4S)- or (1S,4R)-1-amino-4-(hydroxymethyl)-2-cyclopentene according to the formulae 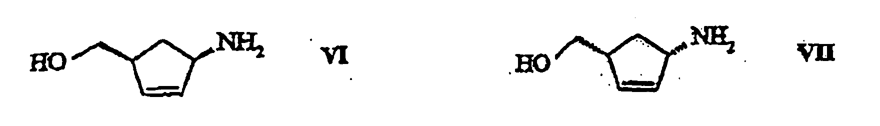 this is transferred, optionally after separation of the enantiomers, in a fourth stage with N-(2-amino-4,6-dichloropyrimidin-5-yl)formamide of the formula 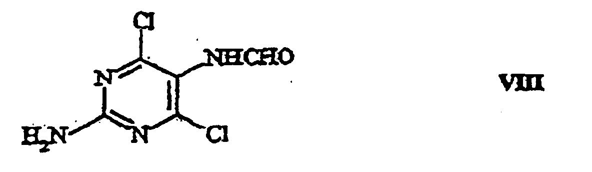 to the (1S,4R)- or (1R,4S)-4-[(2-amino-6-chloro-5-formamido-4-pyrimidinyl)-amino]-2-cyclopentene-1-methanol of the formulae 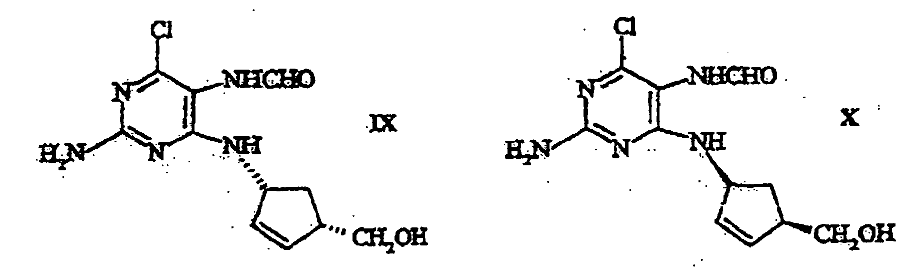 and the latter is cyclised in a fifth stage to the end product according to the formulae I or II.

2. Process according to claim 1, **characterised in that** acylation is carried out in the first stage with a carboxylic acid halide of the general formula 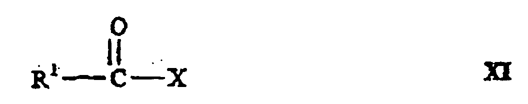 in which R¹ has the given meaning and X means a halogen atom, or with a carboxylic acid anhydride of the general formula 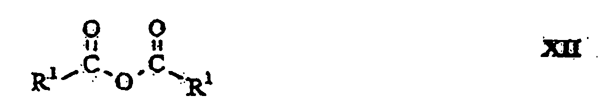 in which R¹ has the given meaning.

3. Process according to one of claims 1 or 2, **characterised in that** acylation is carried out in the first stage in an aprotic solvent.

4. Process according to one of claims 1 to 3, **characterised in that** reduction is carried out in the second stage with an alkali- or earth alkali metal boron hydride, an alkali- or earth alkali metal aluminium hydride or with a vitride.

5. Process according to one of claims 1 to 4, **characterised in that** reduction is carried out in the second stage in a protic solvent.

6. Process according to one of claims 1 to 5, **characterised in that** conversion is carried out in the third stage by means of microorganisms of the type Rhodococcus, Gordona, Arthrobacter, Alcaligenes, Agrobacterium/Rhizobium, Bacillus, Pseudomonas or Alcaligenes/Bordetella.

7. Process according to one of claims 1 to 5, **characterised in that** conversion is carried out in the third stage by means of a penicillin G acylase of microorganisms of the species Bacillus megaterium or Escherichia coli.

8. Process according to one of claims 1 to 7, **characterised in that** conversion is carried out in the third stage at a temperature of 20 to 40°C and a pH of 5 to 9.

9. Process according to one of claims 1 to 8, **characterised in that** conversion is carried out in the fourth stage in the presence of a base.

10. Process according to one of claims 1 to 9, **characterised in that** conversion is carried out in the fourth stage in a protic solvent.

11. Process for the production of (1R,4S)- and/or (1S/4R)-1-amino-4-(hydroxymethyl)-2-cyclopentene derivatives of the general formulae XVI or XVII 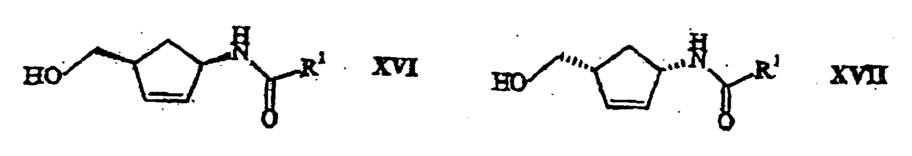 in which R¹ means C₁₋₄-alkyl, C₁₋₄-alkoxy, aryl or aryloxy, **characterised in that** a cyclopentene derivative of the general formula 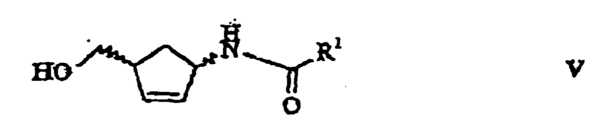 in which R¹ has the given meaning is converted by means of a microorganism that is capable of using cyclopentane derivatives of the general formula V as the sole nitrogen-, the sole carbon- or the sole carbon- and nitrogen source, enzyme extracts thereof, of an enzyme having N-acetylamino alcohol hydrolase activity or of a penicillin G acylase to (1R,4S)- and (1S,4R)-1-amino-4-(hydroxymethyl)-2-cyclopentene according to the formulae 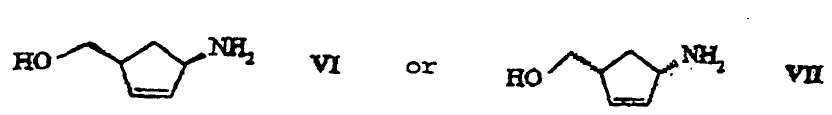 and this is acylised, optionally after separation of the enantiomers, to a compound of the formulae XVI or XVII.

12. (1R,4S)-N-butyryl-1-amino-4-(hydroxymethyl)-2-cyclopentene of the formula 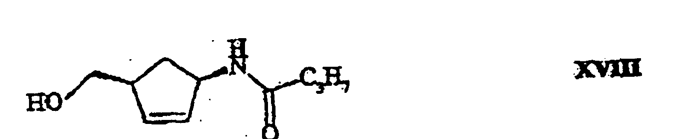 in enantiomer excess.

13. Enantiomers according to claim 12 having an enantiomer excess of at least 80%.

14. Enantiomers according to claim 12 having an enantiomer excess of at least 98%.

## Revendications

1. Procédé de préparation du (1S,4R)- et/ou (1R,4S)-4-(2-amino-6-chloro-9-H-purin-9-yl)-2-cyclopentène-1-méthanol de formule I ou II 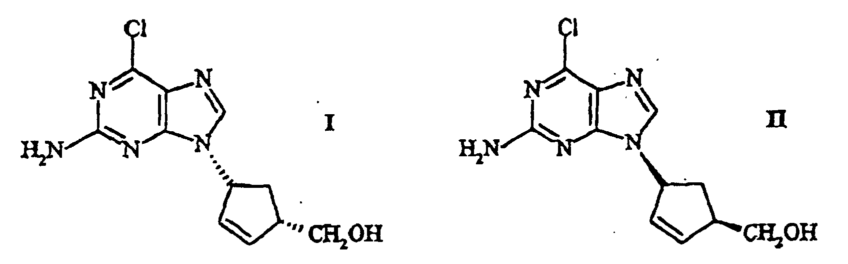 ou de ses sels, **caractérisé en ce que**, dans une première étape, on acyle une (±)-2-azabicyclo[2.2.1]hept-5-én-3-one de formule 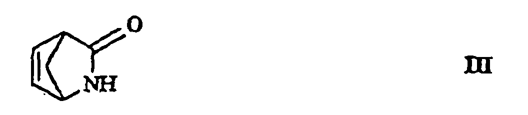 en un dérivé de (±)-2-azabicyclo[2.2.1]hept-5-én-3-one de formule générale 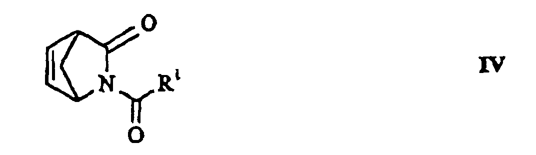 où R¹ représente C₁₋₄-alkyle, C₁₋₄-alcoxy, aryle ou aryloxy, dans une deuxième étape on réduit celui-ci en un dérivé de 1-amino-4-(hydroxyméthyl)-2-cyclopentène de formule générale 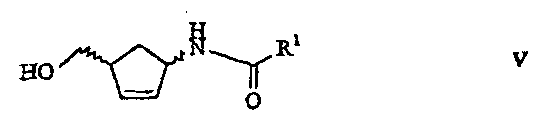 où R¹ a la signification citée, dans une troisième étape on convertit celui-ci au moyen d'un micro-organisme qui est capable d'utiliser des dérivés du cyclopentane de formule générale V comme seule source d'azote, comme seule source de carbone ou comme seule source de carbone et d'azote, d'extraits enzymatiques de celui-ci, d'une enzyme à activité de N-acétylaminoalcool hydrolase ou d'une pénicilline G acylase en (1R,4S)- ou (1S,4R)-1-amino-4-(hydroxyméthyl)-2-cyclopentène selon les formules 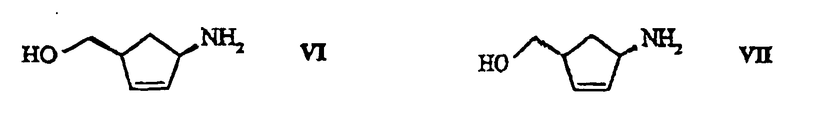 dans une quatrième étape on convertit celui-ci, éventuellement après séparation des énantiomères, avec le N-(2-amino-4,6-dichloropyrimidin-5-yl)formamide de formule 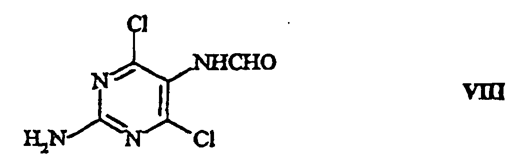 en le (1S,4R)- ou (1R,4S)-4-[(2-amino-6-chloro-5-formamido-4-pyrimidinyl)-amino]-2-cyclopentène-1-méthanol des formules 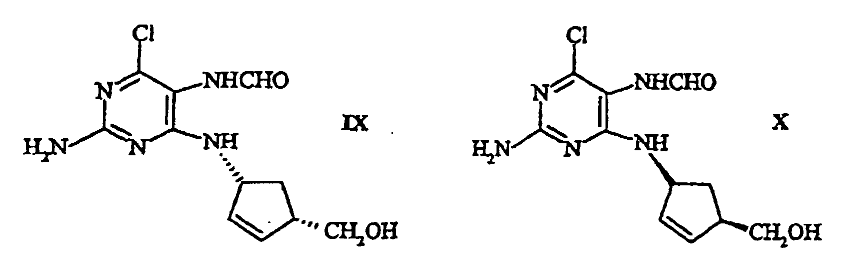 et, dans une cinquième étape, on cyclise ce dernier en le produit final selon la formule I ou II.

2. Procédé selon la revendication 1 **caractérisé en ce que** l'on conduit l'acylation dans la première étape avec un halogénure d'acide carboxylique de formule générale 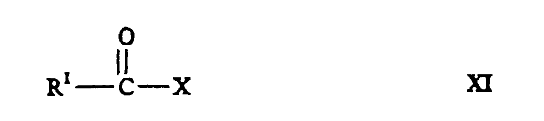 où R¹ a la signification citée et X représente un atome d'halogène, ou avec un anhydride d'acide carboxylique de formule générale 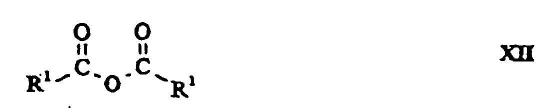 où R¹ a la signification citée.

3. Procédé selon l'une des revendications 1 et 2 **caractérisé en ce que** l'on conduit l'acylation dans la première étape dans un solvant aprotique.

4. Procédé selon l'une des revendications 1 à 3 **caractérisé en ce que** l'on conduit la réduction dans la deuxième étape avec un borohydrure de métal alcalin ou alcalino-terreux, un hydrure d'aluminium et de métal alcalin ou alcalino-terreux ou avec un vitride.

5. Procédé selon l'une des revendications 1 à 4 **caractérisé en ce que** l'on conduit la réduction dans la deuxième étape dans un solvant protique.

6. Procédé selon l'une des revendications 1 à 5 **caractérisé en ce que** l'on conduit la conversion dans la troisième étape au moyen de micro-organismes du genre Rhodococcus, Gordona, Arthrobacter, Alcaligenes, Agrobacterium/Rhizobium, Bacillus, Pseudomonas ou Alcaligenes/Bordetella.

7. Procédé selon l'une des revendications 1 à 5 **caractérisé en ce que** l'on conduit là conversion dans la troisième étape au moyen d'une pénicilline G acylase provenant de micro-organismes des espèces Bacillus megaterium ou Escherichia coli.

8. Procédé selon l'une des revendications 1 à 7 **caractérisé en ce que** l'on conduit la conversion dans la troisième étape à une température de 20 à 40°C et à un pH de 5 à 9.

9. Procédé selon l'une des revendications 1 à 8 **caractérisé en ce que** l'on conduit la conversion dans la quatrième étape en présence d'une base.

10. Procédé selon l'une des revendications 1 à 9 **caractérisé en ce que** l'on conduit la conversion dans la quatrième étape dans un solvant protique.

11. Procédé de préparation de dérivés de (1R,4S)- et/ou (1S,4R)-1-amino-4-(hydroxyméthyl)-2-cyclopentène de formule générale XVI ou XVII 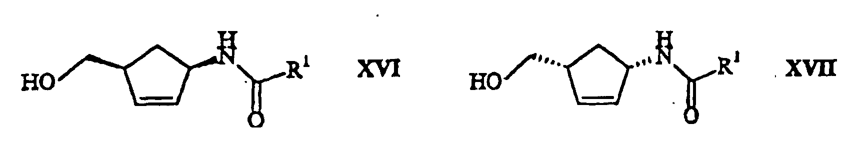 où R¹ représente C₁₋₄-alkyle, C₁₋₄-alcoxy, aryle ou aryloxy, **caractérisé en ce que** l'on convertit un dérivé du cyclopentène de formule générale 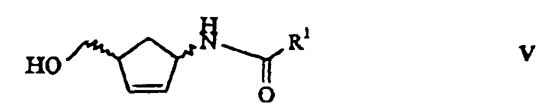 où R¹ a la signification citée, au moyen d'un micro-organisme qui est capable d'utiliser des dérivés du cyclopentane de formule générale V comme seule source d'azote, comme seule source de carbone ou comme seule source de carbone et d'azote, d'extraits enzymatiques de celui-ci, d'une enzyme à activité de N-acétylaminoalcool hydrolase ou d'une pénicilline G acylase en (1R,4S)- ou (1S,4R)-1-amino-4-(hydroxyméthyl)-2-cyclopentène selon les formules 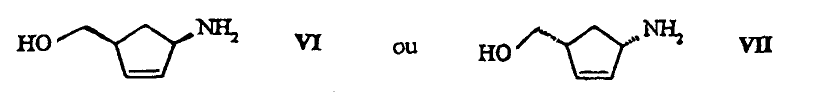 et on acyle celui-ci, éventuellement après séparation des énantiomères, en les composés de formule XVI ou XVII.

12. (1R,4S)-N-butyryl-1-amino-4-(hydroxyméthyl)-2-cyclopentène de formule 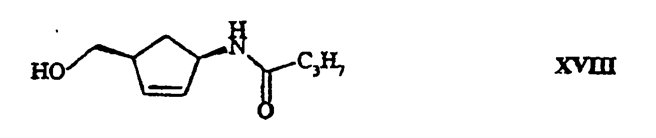 en excès énantiomérique.

13. Enantiomère selon la revendication 12 avec un excès énantiomérique d'au moins 80 %.

14. Enantiomère selon la revendication 12 avec un excès énantiomérique d'au moins 98 %.
